# EUROPEAN PATENT SPECIFICATION

(11) **EP 4 137 124 B1**
(45) Date of publication and mention of the grant of the patent: **25.02.2026**
(21) Application number: 21788226.5
(22) Date of filing: 14.04.2021
(51) Int. Cl.: A61K 9/14, A61K 9/20, A61K 9/48, A61K 31/46, A61P 25/30, A61P 25/20, A61P 25/22, A61P 11/00, A61K 31/506

(54) **SOLID PHARMACEUTICAL PREPARATION, PREPARATION METHOD THEREFOR AND USE THEREOF**
FESTE PHARMAZEUTISCHE ZUBEREITUNG, HERSTELLUNGSVERFAHREN DAFÜR UND VERWENDUNG DAVON
PRÉPARATION PHARMACEUTIQUE SOLIDE, SON PROCÉDÉ DE PRÉPARATION ET SON UTILISATION

(30) Priority: 17.04.2020 CN 202010304917; 29.12.2020 CN 202011594757
(43) Date of publication of application: 22.02.2023
(73) Proprietor: Shanghai Haiyan Pharmaceutical Technology Co., Ltd., Shanghai 201203 (CN); Yangtze River Pharmaceutical Group Co., Ltd., Taizhou, Jiangsu 225321 (CN)
(72) Inventor: JIANG, Taotao, Shanghai 201203 (CN); WANG, Jibiao, Shanghai 201203 (CN); YANG, Han, Shanghai 201203 (CN); LI, Li, Shanghai 201203 (CN); DAN, Zhaoling, Shanghai 201203 (CN); ZHU, Keyi, Shanghai 201203 (CN); ZENG, Zhenya, Shanghai 201203 (CN); SU, Bo, Shanghai 201203 (CN); CHEN, Xi, Shanghai 201203 (CN)
(74) Representative: Zimmermann & Partner Patentanwälte mbB
(86) International application number: PCT/CN2021/087262
(87) International publication number: WO 2021/208976

(56) References cited:
- EP-A1- 3 115 362
- EP-A1- 3 336 088
- WO-A1-2015/131773
- WO-A1-2017/028732
- CN-A- 105 209 468
- KYUNG ROK CHU ET AL: "Effect of particle size on the dissolution behaviors of poorly water-soluble drugs", ARCHIVES OF PHARMACAL RESEARCH, PHARMACEUTICAL SOCIETY OF KOREA, HEIDELBERG, vol. 35, no. 7, 3 August 2012 (2012-08-03), pages 1187 - 1195, XP035094775, ISSN: 1976-3786, DOI: 10.1007/S12272-012-0709-3
- "Pharmaceutics; 1st Edition", 31 January 2014, XI'AN JIAOTONG UNIVERSITY PRESS, CN, ISBN: 978-7-5605-5501-0, article CHEN, WEIWEI : "Dissolution Rate", pages: 350 - 351, XP009531066

## Description

### TECHNICAL FIELD

The invention belongs to the field of pharmaceutical formulation, and in particular relates to a solid pharmaceutical formulation comprising an orexin receptor antagonist compound, a preparation method thereof, and use in the manufacture of a medicament for treating an orexin-associated disease.

### BACKGROUND OF THE INVENTION

Orexin (hypocretin) includes two neuropeptides produced in the hypothalamus: orexin A (OX-A) (a peptide containing 33 amino acids) and orexin B (OX-B) (a peptide containing 28 amino acids) (Sakurai T. et al, Cell, 1998, 92, 573-585). It is discovered that orexin stimulates food consumption in rats, which suggests that these peptides have a physiological role as mediators in central feedback mechanisms regulating feeding behavior (Sakurai T. et al., Cell, 1998, 92, 573- 585). Orexin can regulate the state of sleep and insomnia, potentially proposing a new method for treating narcolepsy or insomniac in patients (Chemelli R.M. et al., Cell, 1999, 98, 437-451). Orexin also plays roles in arousal, motivation, learning and memory (Harris, et al, Trends Neurosci., 2006, 29(10), 571-577). In mammals, two orexin receptors have been cloned and characterized: the orexin-1 receptor and the orexin-2 receptor. They belong to the G protein-coupled receptor superfamily (Sakurai T. et al., Cell, 1998, 92, 573-585), in which the orexin-1 receptor (OX or OX1R) is selective for OX-A, and the orexin-2 receptor (OX2 or OX2R) is capable of binding to OX-A as well as OX-B. It is believed that the physiological roles of orexin are achieved through the expression of one or both of the OX1 receptor and OX2 (two subtypes of orexin receptors).

Orexin receptor can be found in the brain of warm-blooded animals and are associated with disorders such as: depression; anxiety; addiction; obsessive-compulsive disorder; affective neurosis; depressive neurosis; anxiety neurosis; psychotic depression disorder; behavior disorder; mood disorder; sexual dysfunction; psychosexual disorder; gender disorder; schizophrenia; manic depression; insanity; dementia; severe mental retardation and dyskinesia, such as Huntington's disease and Tourette syndrome; eating disorder such as anorexia, bulimia, cachexia, and obesity; addictive eating behavior; binge eating behavior; cardiovascular disease; diabetes; appetite/taste disorder; emesis, vomiting, nausea; asthma; cancer; Parkinson's disease; Cushing's syndrome/disease; basophilic adenomas; prolactinomas; hyperprolactinemia; pituitary gland tumor/adenomas; hypothalamic disorder; inflammatory bowel disease; stomach dysfunction; stomach ulcer; adiposogenital dystrophy; anterior pituitary disorder; pituitary disorder; anterior pituitary hypofunction; anterior pituitary hyperfunction; hypothalamic hypogonadism; Kallmann Syndrome (anosmia, hyposmia); functional or psychogenic amenorrhea; hypopituitarism; hypothalamic hypothyroidism; hypothalamus-adrenal dysfunction; sudden hyperprolactinemia; hypothalamic growth hormone deficiency; sudden growth deficiency; dwarfism; gigantism; acromegaly; disturbed biological and circadian rhythms; sleep disturbances associated with disorders such as insanity, neuropathic pain, and restless legs syndrome; heart and lung disease, acute and congestive heart failure; hypotension; hypertension; urine retention; osteoporosis; angina pectoris; acute myocardial infarction; ischemic or hemorrhagic stroke; arachnoid hemorrhage; ulcers; allergy; benign prostatic hypertrophy; chronic renal failure; kidney disease; impaired glucose tolerance; migraine; hyperalgesia; pain; increased or exaggerated sensitivity to pain, such as hyperalgesia, burning pain and allodynia; acute pain; burning pain; atypical facial pain; neuropathic pain; back pain; complex regional pain syndromes I and II; arthritis pain; sport trauma pain; pain associated with infections such as HIV, post-chemotherapy pain; post-stroke pain; postoperative pain; neuralgia; emesis, nausea, vomiting; conditions associated with visceral pain, such as irritable bowel syndrome and angina; migraine; bladder incontinence, such as urge incontinence; tolerance to narcotic or withdrawal from narcotic; sleep disorder; sleep apnea; narcolepsy; insomnia; parasomnia; jet lag syndrome; and neurodegenerative disorder, including disease classification entities such as disinhibition-dementia-Parkinson's disease-muscular dystrophy syndrome; epilepsy; seizure disorder and other diseases associated with common dysfunction of the orexin system.

EP 3 336 088 A1 discloses crystalline form I, crystalline form II of the compound 5-3 (corresponds to the compound represented by formula (II) of the present application), and its application in the manufacture of medicaments for the treatment or prevention of neurological and psychiatric disorders or orexin-related diseases. As shown in Solubility Test in EP 3 336 088 A1, crystal form I and crystal form II have low solubilities. In addition, EP 3 336 088 A1 does not disclose the particle size of the active ingredient.

CN106414439A discloses a class of piperidine derivatives as the orexin receptor antagonists, which have significant inhibitory effect on OX1 and OX2 GPCR receptor. It is found in the study that when they are prepared into solid pharmaceutical compositions, there are disadvantages of lower dissolution rate and dissolution level, as well as lower bioavailability and longer in vivo half-life, whereas long half-life of drugs for treating insomnia clearly has adverse effects, such as a residual effect on the second day, resulting in adverse consequences for the patients. Therefore, it is necessary to develop it into a solid pharmaceutical dosage form with higher dissolution rate and dissolution level, as well as higher bioavailability and lower half-life, in order to meet wider clinical administration requirements.

### SUMMARY OF THE INVENTION

The invention is as defined in the accompanying claims. The object of the present invention is to provide a solid pharmaceutical formulation comprising the compound of formula I with a good stability, a good dissolution effect and a good bioavailability.

Generally disclosed is a solid pharmaceutical formulation, wherein the solid pharmaceutical formulation comprises an active ingredient, and the active ingredient is a compound represented by formula (I) or a pharmaceutically acceptable salt thereof, or a mixture of both; 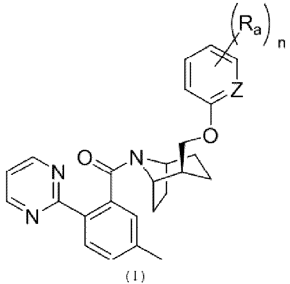
wherein Rₐ is hydrogen, fluorine, chlorine, methyl, ethyl, propyl, isopropyl, methoxy, ethoxy, propoxy or isopropoxy;
Z is N or CR₀; R₀ is hydrogen, halogen or C₁₋₃ alkyl;
n is 0, 1 or 2;
and the particle size of the active ingredient is D90 ≤ 50 µm.

According to the invention, the particle size of the active ingredient is D90 ≤ 35 µm. In another preferred aspect, the particle size of the active ingredient is D90 ≤ 30 µm. In another preferred aspect, the particle size of the active ingredient is D90 ≤ 20µm. In another preferred aspect, the particle size of the active ingredient is D90 ≤ 10µm. In another preferred aspect, the particle size of the active ingredient is D90 = 1µm to 30µm. In another preferred aspect, the particle size of the active ingredient is D90 =1µm to 20µm. In another preferred aspect, the particle size of the active ingredient is D90 = 1 µm to 10 µm or D90 = 10 µm to 20 µm.

According to the invention, the compound represented by formula (I) is a compound of formula (II):

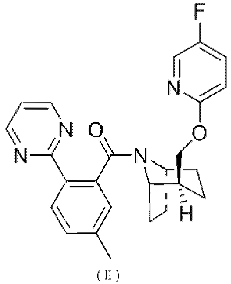

((1S,2R,5S)-2-(((5-fluoropyridin-2-yl)oxy)methyl)-8-azabicyclo[3.2.1]octan-8-yl)(5-methyl-2-(pyrimidin-2-yl)phenyl)methanone. As described in patent CN106414439A, the intracellular calcium signal change is detected by FLIPR and indicated by the IC₅₀ value of the compound to evaluate the inhibitory effect of the compound of formula (II) on OX1 and OX2 GPCR receptors, and the activities thereof are 20 nM (hOX1R) and 36 nM (hOX2R), respectively.

The pharmaceutically acceptable salt of the present invention includes pharmaceutically acceptable acid addition salt and pharmaceutically acceptable base addition salt. The pharmaceutically acceptable acid addition salt refers to a salt formed by an inorganic or organic acid which can retain the biological effectiveness of the free base without other side effects. The inorganic acid salt includes hydrochloride, hydrobromide, sulfate, and phosphate. The organic acid salt includes formate, acetate, propionate, glycolate, gluconate, lactate, oxalate, maleate, succinate, fumarate, tartrate, citrate, glutamate, aspartate, benzoate, mesylate, p-toluenesulfonate, and salicylate. These salts can be prepared by methods known in the art. The pharmaceutically acceptable base addition salt includes a salt of an inorganic base such as sodium salt, potassium salt, calcium salt, and magnesium salt, and includes a salt of an organic base such as ammonium salt, triethylamine salt, lysine salt, and arginine salt. These salts can be prepared by methods known in the art.

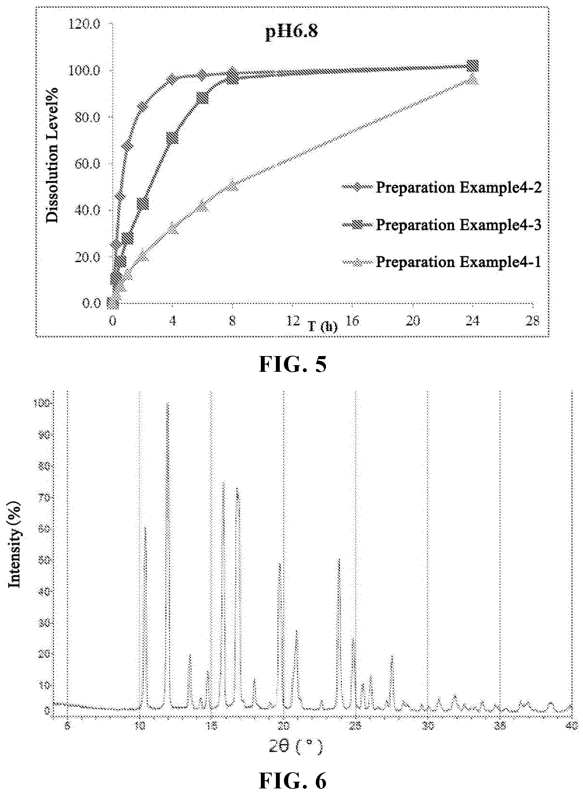

The compound represented by formula (I) and formula (II) of the present invention or the pharmaceutically acceptable salt thereof can be in any form, and the specific form includes amorphous, any crystalline form, hydrate, and solvate. In some embodiments, the compound represented by formula (II) exists in crystalline form A with Cu-Kα radiation XRPD spectrum thereof resolved as follows, and structure thereof shown in FIG. 6.

| **Diffraction Angle 2θ** | **Relative Intensity (%)** | **Diffraction Angle 2θ** | **Relative Intensity (%)** |
|---|---|---|---|
| 10.428 | 60.0 | 25.533 | 8.2 |
| 11.968 | 100.0 | 26.083 | 11.0 |
| 13.542 | 17.8 | 27.133 | 3.1 |
| 14.238 | 3.4 | 27.506 | 17.7 |
| 14.767 | 12.1 | 28.316 | 3.0 |
| 15.851 | 73.9 | 29.557 | 2.2 |
| 16.818 | 72.0 | 30.740 | 4.2 |
| 18.003 | 9.2 | 31.846 | 5.1 |
| 19.087 | 2.1 | 32.550 | 2.0 |
| 19.755 | 47.6 | 33.775 | 3.1 |
| 20.900 | 25.7 | 34.682 | 2.2 |
| 22.652 | 2.9 | 36.422 | 3.4 |
| 23.858 | 49.2 | 36.953 | 3.4 |
| 24.844 | 23.2 | 38.569 | 3.0 |

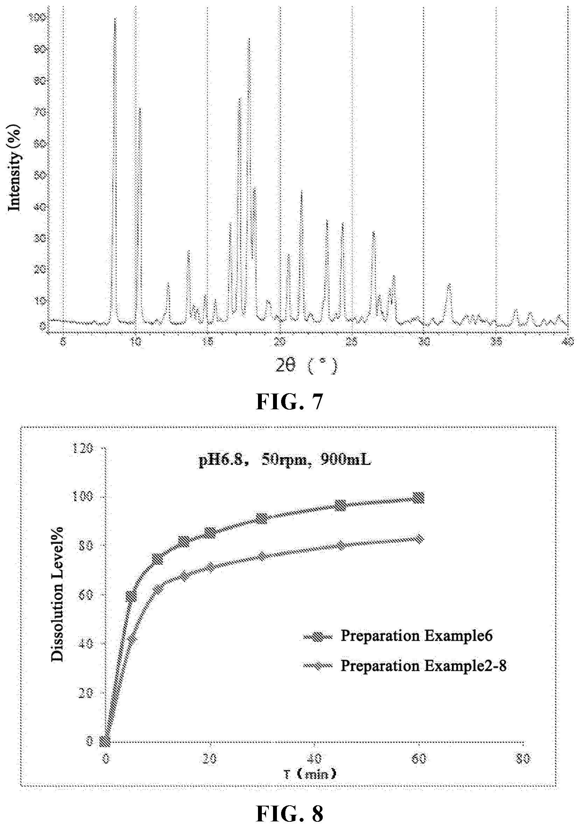

In some embodiments, the compound represented by formula (II) exists in crystalline form B with Cu-Kα radiation XRPD spectrum thereof resolved as follows, and structure thereof shown in FIG. 7.

| **Diffraction Angle2θ** | **Relative Intensity (%)** | **Diffraction Angle2θ** | **Relative Intensity (%)** |
|---|---|---|---|
| 8.611 | 100.0 | 23.953 | 2.6 |
| 10.330 | 70.6 | 24.370 | 32.3 |
| 12.284 | 13.4 | 26.501 | 29.4 |
| 13.703 | 23.9 | 26.915 | 8.7 |
| 14.057 | 5.6 | 27.663 | 11.0 |
| 14.351 | 4.8 | 27.939 | 15.5 |
| 14.848 | 9.2 | 29.499 | 2.6 |
| 15.576 | 7.5 | 30.604 | 2.2 |
| 16.602 | 31.6 | 31.727 | 13.3 |
| 17.250 | 71.3 | 32.969 | 2.6 |
| 17.902 | 90.7 | 33.378 | 2.8 |
| 18.277 | 42.4 | 33.777 | 3.0 |
| 19.149 | 6.5 | 34.825 | 1.6 |
| 20.625 | 21.7 | 36.399 | 5.3 |
| 21.550 | 42.7 | 37.366 | 4.3 |
| 22.124 | 2.3 | 39.357 | 3.3 |
| 23.307 | 33.4 | | |

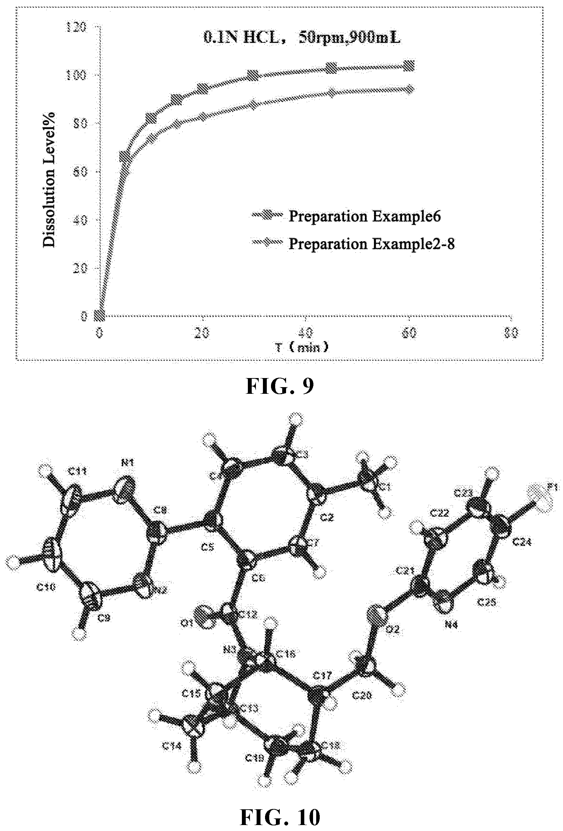

The crystalline form A and the crystalline form B mentioned in the present invention can be prepared and characterized with reference to the method described in the patent CN107709318A. In some embodiments, the compound of formula (II) exists in a crystalline form with a single crystal X-ray diffraction spectrum thereof indicating that the compound of formula (II) has a three-dimensional ellipsoid structure as shown in FIG. 10.

According to the invention, the content of the active ingredient is 1%-15%, more preferably 4%-10%, more preferably 9.5%-10% based on the total dry weight of the solid pharmaceutical formulation.

According to the invention, the solid pharmaceutical formulation further comprises a binder selected from the group consisting of hypromellose, hydroxypropyl cellulose, povidone, sodium alginate, carbopol^{®}, polyvinyl alcohol and a combination thereof. The content of the binder is 0.5%-10%, more preferably 1.5%-3% based on the total dry weight of the solid pharmaceutical formulation.

In another preferred aspect, the binder is selected from the group consisting of hypromellose-E5, hypromellose-K4M, hypromellose-E50, carbopol^{®}, polyvinyl alcohol and a combination thereof.

In another preferred aspect, the binder is hypromellose-E5.

According to the invention, the solid pharmaceutical formulation further comprises a filler selected from the group consisting of microcrystalline cellulose, lactose, cellulose-lactose complex, pre-gelatinized starch, calcium hydrogen phosphate, calcium carbonate and a combination thereof. The content of the filler is 60%-90%, more preferably 73%-85%, more preferably 73%-82.5% based on the total dry weight of the solid pharmaceutical formulation.

In another preferred aspect, the filler is microcrystalline cellulose and lactose.

According to the invention, the solid pharmaceutical formulation further comprises a disintegrant selected from the group consisting of croscarmellose sodium, hypromellose-K4M, crospovidone, sodium carboxymethyl starch and a combination thereof. The content of the disintegrant is 5%-15% based on the total dry weight of the solid pharmaceutical formulation.

In another preferred aspect, the disintegrant is croscarmellose sodium or hypromellose-K4M.

According to the invention, the solid pharmaceutical formulation further comprises a lubricant selected from the group consisting of magnesium stearate, talcum powder, glycerol monostearate, sodium stearyl fumarate and a combination thereof. The content of the lubricant is 0.1%-1%, more preferably 0.4%-0.5%, more preferably 0.48%-0.5% based on the total dry weight of the solid pharmaceutical formulation.

In another preferred aspect, the solid pharmaceutical formulation is a tablet, a capsule, a powder, a granule, a drop pill or a film, preferably a tablet.

In another preferred aspect, the solid pharmaceutical formulation comprises the following components based on the total dry weight of the solid pharmaceutical formulation: a) the active ingredient: ((1S,2R,5S)-2-(((5-fluoropyridin-2-yl)oxy)methyl)-8-azabicyclo[3.2.1]octan-8-yl)(5-methyl-2-(pyrimidin-2-yl)phenyl)methanone, or a pharmaceutically acceptable salt thereof, or a mixture of both, wherein the content of the active ingredient is 1%-15%, more preferably 4%-10%, more preferably 9.5%-10%;
b) the filler selected from the group consisting of microcrystalline cellulose, lactose, cellulose-lactose complex, pre-gelatinized starch, calcium hydrogen phosphate, calcium carbonate and a combination thereof, wherein the content of the filler is 60%-90%, more preferably 73%-85%, more preferably 73%-82.5%;
c) the binder selected from the group consisting of hypromellose, hydroxypropyl cellulose, povidone, sodium alginate, carbopol^{®}, polyvinyl alcohol and a combination thereof, wherein the content of the binder is 0.5%-10%, more preferably 1.5%-3%;
d) the disintegrant selected from the group consisting of croscarmellose sodium, hypromellose-K4M, crospovidone, sodium carboxymethyl starch and a combination thereof, wherein the content of the disintegrant is 5%-15%; and
e) the lubricant selected from the group consisting of magnesium stearate, talcum powder, glycerol monostearate, sodium stearyl fumarate and a combination thereof, wherein the content of the lubricant is 0.1%-1%, more preferably 0.4%-0.5%, more preferably 0.48%-0.5%;
wherein the particle size of the active ingredient is D90 ≤ 35µm.

In another preferred aspect, the particle size of the active ingredient is D90 ≤ 30 µm. In another preferred aspect, the particle size of the active ingredient is D90 ≤ 20 µm. In another preferred aspect, the particle size of the active ingredient is D90 ≤ 10µm. In another preferred aspect, the particle size of the active ingredient is D90 = 1µm to 30µm.In another preferred aspect, the particle size of the active ingredient is D90 = 1 µm to 20 µm. In another preferred aspect, the particle size of the active ingredient is D90 = 1 µm to 10 µm or D90 = 10 µm to 20 µm.

In another preferred aspect, the solid pharmaceutical formulation is a tablet.

In another preferred aspect, the content of the active ingredient is 5 mg - 100 mg. In another preferred aspect, the content of the active ingredient is 10 mg - 50 mg. In another preferred aspect, the content of the active ingredient is 10 mg, 20 mg or 40 mg.

In another preferred aspect, the binder is selected from the group consisting of hypromellose-E5, hypromellose-K4M, hypromellose-E50, carbopol^{®}, polyvinyl alcohol and a combination thereof.

In another preferred aspect, the binder is hypromellose-E5.

In another preferred aspect, the filler is microcrystalline cellulose and lactose.

In another preferred aspect, the disintegrant is croscarmellose sodium or hypromellose-K4M.

In another preferred aspect, the solid pharmaceutical formulation comprises the following components based on the total dry weight of the solid pharmaceutical formulation: a) the active ingredient: ((1S,2R,5S)-2-(((5-fluoropyridin-2-yl)oxy)methyl)-8-azabicyclo[3.2.1]octan-8-yl)(5-methyl-2-(pyrimidin-2-yl)phenyl)methanone, or a pharmaceutically acceptable salt thereof, or a mixture of both, wherein the content of the active ingredient is 4%-10%;
b) the microcrystalline cellulose with a content of 24%-27.5%;
c) the lactose with a content of 48.5%-56.5%;
d) the hypromellose-E5 with a content of 1.5%-3%;
e) the croscarmellose sodium or hypromellose-K4M with a content of 5%-15%; and
f) the magnesium stearate with a content of 0.4%-0.5%;
wherein the particle size of the active ingredient is D90 ≤ 35 µm, or D90 ≤ 30 µm, or D90 ≤ 20 µm, or D90 ≤ 10 µm, or D90 = 1 µm to 30 µm, or D90 = 1 µm to 20 µm, or D90 =1 µm to 10 µm, or D90 = 10 µm to 20 µm.

In another preferred aspect, the solid pharmaceutical formulation comprises the following components based on the total dry weight of the solid pharmaceutical formulation:
a) the active ingredient: ((1S,2R,5S)-2-(((5-fluoropyridin-2-yl)oxy)methyl)-8-azabicyclo[3.2.1]octan-8-yl)(5-methyl-2-(pyrimidin-2-yl)phenyl)methanone, or a pharmaceutically acceptable salt thereof, or a mixture of both, wherein the content of the active ingredient is 9.5%-10%;
b) the microcrystalline cellulose with a content of 24%-27.5%;
c) the lactose with a content of 48.5%-56.5%;
d) the hypromellose-E5 with a content of 1.5% to 3%;
e) the croscarmellose sodium or hypromellose-K4M with a content of 5% to 15%; and
f) the magnesium stearate with a content of 0.48% to 0.5%;
wherein the particle size of the active ingredient is D90 = 1 µm to 30 µm, or D90 = 1 µm to 20 µm, or D90 = 1 µm to 10 µm, or D90 = 10 µm to 20 µm, and the solid pharmaceutical formulation is a tablet.

In another preferred aspect, the lactose is 200 mesh, 100 mesh, or 50 mesh. More preferably, the lactose is 200 mesh.

In another preferred aspect, the filler is microcrystalline cellulose and lactose. The weight ratio of microcrystalline cellulose to lactose is (24-27.5):(48.5-56.5).

In another preferred aspect, the solid pharmaceutical formulation comprises any one of tablets in the preparation example.

In another aspect, the present invention provides a unit dosage form, based on a total weight of the unit dosage form, the unit dosage form comprises: about 10 mg, about 20 mg, or about 40 mg of an active ingredient, wherein the active ingredient is a compound of formula (II); about 25 mg to about 150 mg of microcrystalline cellulose (for example, microcrystalline cellulose PH101); about 50 mg to about 300 mg of lactose (for example, Granulac^{®} 200); about 1 mg to about 15 mg of hypromellose (for example, hypromellose E5); about 10 mg to about 50 mg of croscarmellose sodium (for example, croscarmellose sodium SD711) or hypromellose (for example, HPMC-K4M); and about 0.5 mg to about 3 mg of magnesium stearate, wherein the particle size of the active ingredient is D90 = 1 µm to 20 µm. In some embodiments, the particle size of the active ingredient is D90 = 1 µm to 10 µm or D90 = 10 µm to 20 µm.

In another aspect, the present invention provides a unit dosage form, based on a total weight of the unit dosage form, the unit dosage form comprises: about 10 mg of an active ingredient, wherein the active ingredient is a compound of formula (II); about 25 mg to about 26 mg of microcrystalline cellulose (for example, microcrystalline cellulose PH101); about 51 mg of lactose (for example, Granulac^{®} 200); about 3 mg of hypromellose (for example, hypromellose E5); about 10 mg of croscarmellose sodium (for example, croscarmellose sodium SD711); and about 0.5 mg of magnesium stearate, wherein the particle size of the active ingredient is D90 = 1 µm to 20 µm. In some embodiments, the particle size of the active ingredient is D90 = 1 µm to 10 µm or D90 = 10 µm to 20 µm.

In another aspect, the present invention provides a unit dosage form, based on a total weight of the unit dosage form, the unit dosage form comprises: about 20 mg of an active ingredient, wherein the active ingredient is a compound of formula (II); about 51 mg of microcrystalline cellulose (for example, microcrystalline cellulose PH101); about 102 mg of lactose (for example, Granulac^{®} 200); about 6 mg of hypromellose (for example, hypromellose E5); about 20 mg of croscarmellose sodium (for example, croscarmellose sodium SD711); and about 1 mg of magnesium stearate, wherein the particle size of the active ingredient is D90 = 1 µm to 20 µm. In some embodiments, the particle size of the active ingredient is D90 = 1 µm to 10 µm or D90 = 10 µm to 20 µm.

In another aspect, the present invention provides a unit dosage form, based on a total weight of the unit dosage form, the unit dosage form comprises: about 40 mg of an active ingredient, wherein the active ingredient is a compound of formula (II); about 102 mg of microcrystalline cellulose (for example, microcrystalline cellulose PH101); about 204 mg of lactose (for example, Granulac^{®} 200); about 12 mg of hypromellose (for example, hypromellose E5); about 40 mg of croscarmellose sodium (for example, croscarmellose sodium SD711); and about 2 mg of magnesium stearate, wherein the particle size of the active ingredient is D90 = 1 µm to 20 µm. In some embodiments, the particle size of the active ingredient is D90 = 1 µm to 10 µm or D90 = 10 µm to 20 µm.

In another aspect, the present invention provides a unit dosage form, based on a total weight of the unit dosage form, the unit dosage form comprises 8 mg to 12 mg of an active ingredient, wherein the active ingredient is a compound of formula (II); 20 mg to 30 mg of microcrystalline cellulose (for example, microcrystalline cellulose PH101); 46 mg to 56 mg of lactose (for example, Granulac^{®} 200); 2 mg to 4 mg of hypromellose (for example, hypromellose E5); 5 mg to 15 mg of croscarmellose sodium (for example, croscarmellose sodium SD711); and 0.3 mg to 0.7 mg of magnesium stearate, wherein the particle size of the active ingredient is D90 = 1 µm to 20 µm. In some embodiments, the particle size of the active ingredient is D90 = 1 µm to 10 µm or D90 = 10 µm to 20 µm.

In another aspect, the present invention provides a unit dosage form, based on the total weight of the unit dosage form, the unit dosage form comprises: 18 mg to 22 mg of an active ingredient, wherein the active ingredient is a compound of formula (II); 46 mg to 56 mg of microcrystalline cellulose (for example, microcrystalline cellulose PH101); 97 mg to 107 mg of lactose (for example, Granulac^{®} 200); 5 mg to 7 mg of hypromellose (for example, hypromellose E5); 15 mg to 25 mg of croscarmellose sodium (for example, croscarmellose sodium SD711); and 0.8 mg to 1.2 mg of magnesium stearate, wherein the particle size of the active ingredient is D90 = 1 µm to 20 µm. In some embodiments, the particle size of the active ingredient is D90 = 1 µm to 10 µm or D90 = 10 µm to 20 µm.

In another aspect, the present invention provides a unit dosage form, based on a total weight of the unit dosage form, the unit dosage form comprises: 38 mg to 42 mg of an active ingredient, wherein the active ingredient is a compound of formula (II); 97 mg to 107 mg of microcrystalline cellulose (for example, microcrystalline cellulose PH101); 199 mg to 209 mg of lactose (for example, Granulac^{®} 200); 11mg to 13mg of hypromellose (for example, hypromellose E5); 35 mg to 45 mg of croscarmellose sodium (for example, croscarmellose sodium SD711); and 1.8 mg to 2.2 mg of magnesium stearate, wherein the particle size of the active ingredient is D90 = 1 µm to 20 µm. In some embodiments, the particle size of the active ingredient is D90 = 1 µm to 10 µm or D90 = 10 µm to 20 µm.

In some embodiments, in any one of above unit dosage forms provided by the present invention, the compound of formula (II) as the active ingredient may exist in any form, including amorphous, any crystalline form, hydrate, and solvate. In some embodiments, the compound of formula (II) as the active ingredient exists in amorphous, crystalline form A or crystalline form B. In some embodiments, the compound of formula (II) as the active ingredient exists in crystalline Form A.

In some embodiments, the above unit dosage form is a tablet or a capsule. In some embodiments, the above unit dosage form is a tablet. In some embodiments, the above unit dosage form is a tablet and further comprises a coating.

The tablet can be prepared by methods including conventional compression, wet granulation or dry granulation. In some embodiments, the dosage form of the present invention is a tablet prepared by a wet granulation process. The tablet may also comprise one or more surface coatings, such as a clear coating and/or a colored coating. Various coatings and their application methods are known in the art, including those disclosed in Remington's Pharmaceutical Sciences (17^{th} Edition, Mack Publishing Company, Easton, Pa., 1985). When an appropriate amount of coating is present, the weight of the tablet will usually increase by 2% to 3%, so the weight of the tablet can be generally between about 50 mg and about 1000 mg. In some embodiments, the weight of the tablet is about 100 mg, about 150 mg, about 200 mg, about 250 mg, about 400 mg, about 500 mg, about 600 mg, about 700 mg, about 800 mg, or about 900 mg, depending on the dosage required for the therapeutic use.

The expression of "about" used before weight value in the above unit dosage forms of the present invention means a range value of ±10 mg, or ±5 mg, or ±2 mg, or ±1 mg, or ±0.5 mg, or ±0.2 mg, or ±0.1 mg.

The particle size D90 of the active ingredient in the present invention refers to the particle size when the cumulative particle size distribution percentage of a sample reaches 90%.

Film coatings useful in the formulations of the present invention are known in the art and typically comprise polymers (usually cellulosic polymers), colorants and plasticizers. Additional ingredients such as sugars, flavoring agents, oils and lubricants can be comprised in the film coating formulation to impart certain properties to the film coating. The compositions and formulations herein can also be combined and processed into solids and then placed in capsule forms such as gelatin capsules.

It should be understood that some components of the formulations of the present invention may have multiple functions. For example, a given component can be used as a filler or a disintegrant. In some of such cases, the function of the given component may be considered singular, although its properties may allow for multi-functionality.

In the present invention, lactose can be selected from commercially available lactose suitable for the pharmaceutical field, including Flow100, Granulac^{®} 200, Tableffose 100, and Spherolac^{®} 100. The microcrystalline cellulose can be selected from commercially available microcrystalline cellulose suitable for the pharmaceutical field, including pH101, pH102, pH301, pH302, KG1000, KG802, UF702, and UF711. The pre-gelatinized starch (also known as modified starch) can be selected from commercially available pre-gelatinized starch suitable for the pharmaceutical field, including Starch 1500^{®}, and PC10. The hypromellose can be selected from commercially available hypromellose suitable for the pharmaceutical field, including HPMC E3, HPMC E5, HPMC K4M, and HPMC E15. The povidone can be selected from commercially available povidone suitable for the pharmaceutical field, including povidone K30 and povidone K90. The crospovidone can be selected from commercially available crospovidone suitable for the pharmaceutical field, including PVPP XL-10, PVPP VL-10 and PVPP XL. The croscarmellose sodium can be selected from commercially available croscarmellose sodium suitable for the pharmaceutical field, including RC-A591NF, and SD-711.

The second aspect of the present invention provides a method for preparing a tablet of the claimed formulation, the method comprises the following steps:
(a) performing wet granulation after mixing an active ingredient particle, a filler, a binder and a first disintegrant;
(b) drying a resulting product obtained in step (a);
(c) dry-blending a resulting product obtained in step (b), a second disintegrant and a lubricant; and
(d) compressing a resulting product obtained in step (c) into the tablet;

wherein the active ingredient is ((1S,2R,5S)-2-(((5-fluoropyridin-2-yl)oxy)methyl)-8-azabicyclo[3.2.1]octan-8-yl)(5-methyl-2-(pyrimidin-2-yl)phenyl)methanone, or a pharmaceutically acceptable salt thereof, or a mixture of both;
the particle size of the active ingredient particle is D90 ≤ 35 µm.

The first disintegrant and the second disintegrant can be the same or different. In another preferred aspect, the particle size of the active ingredient particles is D90 ≤ 30 µm. In another preferred aspect, the particle size of the active ingredient particles is D90 ≤ 20 µm. In another preferred aspect, the particle size of the active ingredient particles is D90 ≤ 10 µm. In another preferred aspect, the particle size of the active ingredient particles is D90 = 1 µm to 30 µm. In another preferred aspect, the particle size of the active ingredient particles is D90 = 1 µm to 20 µm. In another preferred aspect, the particle size of the active ingredient particles is D90 = 1 µm to 10 µm. In another preferred aspect, the particle size of the active ingredient particles is D90 = 10 µm to 20 µm.

The filler is selected from the group consisting of microcrystalline cellulose, lactose, cellulose-lactose complex, pre-gelatinized starch, calcium hydrogen phosphate, calcium carbonate and a combination thereof.

The binder is selected from the group consisting of hypromellose, hydroxypropyl cellulose, povidone, sodium alginate, carbopol^{®}, polyvinyl alcohol and a combination thereof.

The first disintegrant and the second disintegrant are each independently selected from the group consisting of croscarmellose sodium, hypromellose-K4M, crospovidone, sodium carboxymethyl starch and a combination thereof.

The lubricant is selected from the group consisting of magnesium stearate, talcum powder, glycerol monostearate, sodium stearyl fumarate and a combination thereof.

In another preferred aspect, based on the total dry weight of the mixture obtained in step (c), the content of the active ingredient is 4%-10%.

In another preferred aspect, based on the total dry weight of the mixture obtained in step (c), the content of the filler is 73%-82.5%.

In another preferred aspect, based on the total dry weight of the mixture obtained in step (c), the content of the binder is 1.5%-3%.

In another preferred aspect, based on the dry weight of all components in step (a), the content of the first disintegrant is 2%-10%, more preferably 5%-6%; based on the dry weight of all components in step (c), the content of the second disintegrant is 5%-10%.

In another preferred aspect, based on the total dry weight of all components in step (c), the content of the lubricant is 0.1%-1%, more preferably 0.4%-0.5%, more preferably 0.48%-0.5%.

In another preferred aspect, the method further comprises step (e): coating a product of step (d).

In another preferred aspect, a coating material in step (e) is a stomach-soluble film coating premix, and the concentration of the coating material is 10%-20%.

In another aspect, the present invention also provides a product prepared by the method described herein.

The third aspect of the present invention provides the solid pharmaceutical formulation described in the first aspect of the present invention for use in treating an orexin-associated disease.

More preferably, the orexin-associated diseases include insomnia, chronic obstructive pulmonary disease, obstructive sleep apnea, somnolence, anxiety, obsessive-compulsive disorder, panic, nicotine dependence or eating disorder.

### DESCRIPTION OF THE DRAWINGS

FIG. 1 is a graph showing the dissolution curves of the formulations of Preparation Examples 1-1, 1-2, 1-3 and 1-4.
FIG. 2 is a graph showing the dissolution curves of the formulations of Preparation Examples 2-1, 2-2, 2-6 and 2-7.
FIG. 3 is a graph showing the dissolution curves of the formulations of Preparation Examples 2-1, 2-2, 2-3, 2-4 and 2-5.
FIG. 4 is a graph showing the dissolution curves of the formulations of Preparation Examples 3, 2-1 and 2-4.
FIG. 5 is a graph showing the dissolution curves of the formulations of Preparation Examples 4-1, 4-2 and 4-3.
FIG. 6 is an XRPD spectrum using Cu-Kα radiation of the compound of formula (II) in crystalline form A.
FIG. 7 is an XRPD spectrum using Cu-Kα radiation of the compound of formula (II) in crystalline form B.
FIG. 8 is a graph showing the in vitro dissolution curves of the formulations of Preparation Examples 2-8 and Preparation Example 6, using phosphate buffer at pH 6.8 as the dissolution medium.
FIG. 9 is a graph showing the in vitro dissolution curves of the formulations of Preparation Examples 2-8 and Preparation Example 6, using 0.1 N HCl as the dissolution medium.
FIG. 10 is a three-dimensional ellipsoid structure of the single crystal of the compound of formula (II).

### Preparation method

The solid pharmaceutical formulation of the present invention can be prepared by methods well known in the art. For example, in the case of granule, the compound represented by formula (II) or a pharmaceutically acceptable salt thereof and an excipient, a binder, a disintegrant, a wetting agent and the like can be mixed, and subjected to stirring granulation, extrusion granulation, rotary granulation, one-step spray granulation and the like, or direct dry granulation as required to prepare the granule. In addition, the granule can also be prepared by applying medicine to pellets. In addition, granulation and grinding can also be carried out as required. Furthermore, excipients, disintegrants, binders, antioxidants, colorants and the like can be further added to the above granule for tableting.

For further illustration, the uncoated tablet (plain tablet) or the coated tablet of the present invention can be prepared by the following preparation process by changing the added amount or the corresponding component according to different prescriptions. The preparation process includes: grinding the active pharmaceutical ingredient, sieving the auxiliary material, weighing, mixing and granulating, wet granule sizing, drying, dry granule sizing, final mixing, tableting, and coating (required when preparing a coated tablet).

Grinding the active pharmaceutical ingredient: the particle size of qualified active pharmaceutical ingredient (the compound of formula (II), the pharmaceutically acceptable salt thereof, or the mixture thereof) should be 120-150 µm, which can be achieved by adjusting different parameters of grinding equipment. (1) Particle size control D90 ≤ 10 µm, D90 ≤ 20µm or D90 ≤ 30µm: pass the qualified active pharmaceutical ingredient (the compound of formula (II), the pharmaceutically acceptable salt thereof, or the mixture thereof) through a sieve, then add into a jet mill for grinding, control the particle size of the ground active pharmaceutical ingredient by controlling the rotary speed of the feeder at 100 to 500 rpm, adjusting the feed pressure to 3 to 7 bar and the grinding pressure to 2 to 7 bar, and then measure the particle size distribution by a laser particle size distribution analyzer, in which the particle size distribution should meet the particle size D90 ≤ 10µm, D90 ≤ 20µm, or D90 ≤ 30µm; or (2) particle size control D90 ≤ 35 µm : taking the qualified active pharmaceutical ingredient (of formula (II)), grinding by a laboratory shear pulverizer, grinding 3 minutes with an interval of 5 minutes, after grinding for a certain period of time, measuring the particle size of the active pharmaceutical ingredient, in which the particle size distribution measured by a laser particle size distribution analyzer should meet D90 ≤ 35 µm.

Sieving the auxiliary material: obtain a qualified auxiliary material, lactose (Granulac^{®} 200 mesh).

Weighing: weigh the active pharmaceutical ingredient (ground), microcrystalline cellulose (PH101), lactose (Granulac^{®} 200 mesh) (sieved), hypromellose-E5, croscarmellose sodium or hypromellose-K4M (with a disintegrant added).

Mixing and granulating: pulping, i.e., binder preparation: weigh 300g of purified water, add 30 g of hypromellose-E5 while stirring, keep stirring until dissolved to obtain a 10% hypromellose-E5 aqueous solution, pass through a 60-mesh sieve and reserve. Mixing, i.e.: add the active pharmaceutical ingredient (ground) and lactose (sieved) into a wet mixing granulator successively to start stirring and mixing with a stirring speed of 300-500 rpm (for example, 300 rpm or 400 rpm), a chopping speed of 350-400 rpm or 400-500 rpm (for example, 400 rpm), and a stirring time of 300 seconds; open the pot cover, add croscarmellose sodium or hypromellose-K4M (with a disintegrant added), microcrystalline cellulose (PH101) into the pot successively to start stirring and mixing with a stirring speed of 300-500 rpm (for example, 350 rpm or 400 rpm), a chopping speed of 400-500 rpm (preferably 400 rpm), and a stirring time of 600 seconds. Preparing damp mass, the whole process of which is divided into two stages, a first stage: open the wet mixing granulator with pre-set parameters, set the stirring speed to 350-500 rpm (for example, 350 rpm), and the cutting speed to 1000-1500 rpm (for example, 1000 rpm), run for 10 seconds, then slowly add all of 10% (w/w) hypromellose-E5 aqueous binder into the wet mixing granulator, after adding the binder solution, homogenize the obtained wet granules with stirring paddle and chopper until no obvious agglomerates exist, in which the granulation time (slurry adding time) is ≤300 seconds (for example, 60s); and a second stage: set the stirring speed to 500 rpm or 350 rpm, and the cutting speed to 1500 rpm or 1000 rpm, start stirring and cutting at the same time, and continue stirring and cutting for 60s to prepare suitable wet granules.

Wet granule sizing: subject the resulting wet granules to granule sizing by passing through a 18-mesh stainless steel sieve in a swing granule sizing machine or to manually wet granule sizing by passing through a 20-mesh sieve.

Drying: spread the wet granules after granule sizing evenly in a baking tray, in which the thickness of spread wet granules in the tray should be 1.5 cm ± 0.5 cm, put the baking tray with the spread wet granules into an oven to start drying with a drying temperature of 65.0 ° C ± 5.0°C. Turn the spread wet granules in the tray over and measure the moisture content of the granules for every 30 minutes of drying until the drying end point when the moisture content of the granules after drying ≤ 2.0%.

Dry granule sizing: subject the dried granules to granule sizing by passing through a 20-mesh stainless steel sieve in a swing granule sizing machine.

Final mixing: add croscarmellose sodium or hypromellose-K4M (with a disintegrant added), the granules after dry granule sizing and magnesium stearate into a mixer at the same time and mix for 300 seconds with a mixing speed of 16 rpm. After final mixing, the total moisture content of the granules should be ≤ 3.0%.

Tableting: obtain the final mixed granules of the active pharmaceutical ingredient, and subject to tableting by a rotary tableting machine.

Coating: coat the plain tablets obtained by tableting to obtain a desired coating weight gain of 2% to 3%. The stomach-soluble film coating premix is selected as the coating material, and the concentration of the coating solution is 15%. The specific preparation method comprises: taking 30 g of coating powder, adding into 200 g of purified water, and stirring to disperse evenly to obtain the coating solution (formulated according to 200% of the weight gain at 3%), and then coating the tablets by a high-efficiency coater. Coating can be carried out by using the following process parameters.

| | |
|---|---|
| Inlet air temperature (°C) | 55.0-65.0 |
| Outlet air temperature (°C) | 45.0-50.0 |
| Heating (°C) | 70.0-75.0 |
| Air volume (m³_{/}h) | 80.0-85.0 |
| Flow rate (g/min) | 85-90 |
| Main engine rotary speed (rpm) | 3.0-5.5 |
| Spray gun pressure (bar) | 0.11 or 0.9 |

### DETAILED DESCRIPTION OF THE INVENTION

The present invention will be further described below in conjunction with specific examples. In the following examples, the experimental methods without specific conditions are usually in accordance with conventional conditions, or in accordance with the conditions suggested by the manufacturer. Percentages and parts are weight percentages and weight parts unless otherwise specified. The experimental materials and reagents used in the following examples can be obtained from commercial sources unless otherwise specified.

The single crystal structure is tested using a D8 Venture X-ray single crystal diffractometer, light source: Cu target, X-ray: Cu-Kα (=1.54178Å), detector: CMOS surface detector, resolution: 0.8 Å, current and voltage: 50kV, 1.2mA, exposure time: 10s, distance from the surface detector to the sample: 40mm, test temperature: 150(2)K.

Unless otherwise stated, the weight percentages set forth for the active ingredient, the filler component, the binder component, the disintegrant component, and the lubricant component of the solid pharmaceutical formulation disclosed herein are the percentage of each component in the final solid pharmaceutical formulation without any surface coverings, such as a tablet coating (e.g., any clear coating or colored coating) or a capsule. The calculation of the weight percentages of the active ingredient, the filler component, the binder component, the disintegrant component and the lubricant component may slightly change, since the coated tablet in the following specific examples include the weight of the coating. Unless otherwise stated, the active ingredient of the compound of formula (II) exists in crystalline form A, the microcrystalline cellulose is microcrystalline cellulose PH101, the lactose is lactose Granulac^{®} 200 mesh, and the croscarmellose sodium is croscarmellose sodium SD711 in the specific formulations in the following preparation examples.

### Preparation Example 1-1 Uncoated plain tablet formulation

Tablet formulation comprising the compound of formula (II) (mass percent):

| | |
|---|---|
| compound of formula (II) | 4% |
| microcrystalline cellulose | 26.16% |
| lactose | 56.32% |
| hypromellose-E5 | 3.04% |
| croscarmellose sodium | 10% |
| magnesium stearate | 0.48% |

The compound of formula (II) was passed through a 20-mesh sieve, and then the sieved active pharmaceutical ingredient was added into a jet mill and ground under the grinding pressure of 6 to 7 bar, the inlet pressure of 7 to 8 bar, the feed pressure of 6 to 7 bar, and the feed rotary speed of 100 to 150 rpm. After the grinding was completed, samples were taken and tested, and the particle size distribution was measured by a laser particle size distribution analyzer, showing the particle size D90 ≤ 10µm (D90 = 7.461 µm). The compound of formula (II) with the particle size of D90 ≤ 10 µm (D90 = 7.461 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted to prepare uncoated plain tablets having a specification of 500 mg.

### Preparation Example 1-2 Uncoated plain tablet formulation

Tablet formulation comprising the compound of formula (II) (mass percent):

| | |
|---|---|
| compound of formula (II) | 4% |
| microcrystalline cellulose | 26.16% |
| lactose | 56.32% |
| hypromellose-E5 | 3.04% |
| croscarmellose sodium | 10% |
| magnesium stearate | 0.48% |

The compound of formula (II) was passed through a 20-mesh sieve, and then the sieved active pharmaceutical ingredient was added into a jet mill and ground under the grinding pressure of 4 to 6 bar, the inlet pressure of 6 to 7 bar, the feed pressure of 5 to 6 bar, and the feed rotary speed of 200 to 300 rpm. Samples were taken and tested, and the particle size distribution was measured by a laser particle size distribution analyzer, showing the particle size D90 ≤ 20µm (D90 = 17.89 µm).The compound of formula (II) with D90 ≤ 20 µm (D90 = 17.89 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted to prepare uncoated plain tablets having a specification of 500 mg.

### Preparation Example 1-3 Uncoated plain tablet formulation

Tablet formulation comprising the compound of formula (II) (mass percent):

| | |
|---|---|
| compound of formula (II) | 4% |
| microcrystalline cellulose | 26.16% |
| lactose | 56.32% |
| hypromellose-E5 | 3.04% |
| croscarmellose sodium | 10% |
| magnesium stearate | 0.48% |

The compound of formula (II) was passed through a 20-mesh sieve, and then the sieved active pharmaceutical ingredient was added into a jet mill and ground under the grinding pressure of 2 to 4 bar, the inlet pressure of 4 to 5 bar, the feed pressure of 3 to 4 bar, and the feed rotary speed of 300 to 400 rpm. After the grinding was completed, samples were taken and tested, and the particle size distribution was measured by a laser particle size distribution analyzer, showing the particle size D90 ≤ 35µm (D90 = 30.7 µm). The compound of formula (II) with D90 ≤ 35 µm (D90 = 30.7 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted to prepare uncoated plain tablets having a specification of 500 mg.

### Preparation Example 1-4 Uncoated plain tablet formulation as reference example

Tablet formulation comprising the compound of formula (II) (mass percent):

| | |
|---|---|
| compound of formula (II) | 4% |
| microcrystalline cellulose | 26.16% |
| lactose | 56.32% |
| hypromellose-E5 | 3.04% |
| croscarmellose sodium | 10% |
| magnesium stearate | 0.48% |

After the compound of formula (II) was passed through a 20-mesh sieve, the particle size distribution was measured by a laser particle size distribution analyzer, showing the particle size D90 ≤ 150µm (D90 = 144.5 µm). The compound of formula (II) with the particle size of D90 ≤ 150 µm (D90 = 144.5 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted to prepare uncoated plain tablets having a specification of 500 mg.

### Preparation Example 2-1 Coated tablet formulation

Tablet formulation comprising the compound of formula (II) (mass percent):

| | |
|---|---|
| compound of formula (II) | 9.76% |
| microcrystalline cellulose | 24.88% |
| lactose | 49.76% |
| hypromellose-E5 | 2.93% |
| croscarmellose sodium | 9.76% |
| magnesium stearate | 0.49% |
| film coating premix | 2.44% |

The compound of formula (II) was passed through a 20-mesh sieve, and then the sieved active pharmaceutical ingredient was added into a jet mill and ground under the grinding pressure of 4 to 6 bar, the inlet pressure of 6 to 7 bar, the feed pressure of 5 to 6 bar, and the feed rotary speed of 200 to 300 rpm. Samples were taken and tested, and the particle size distribution was measured by a laser particle size distribution analyzer, showing the particle size D90 ≤ 20µm (D90 = 17.89 µm). The compound of formula (II) with the particle size of D90 ≤ 20 µm (D90 = 17.89 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted and coated with a stomach-soluble film coating premix to prepare tablets having a specification of 205 mg.

### Preparation Example 2-2 Coated tablet formulation

Tablet formulation comprising the compound of formula (II) (mass percent):

| | |
|---|---|
| compound of formula (II) | 9.76% |
| microcrystalline cellulose | 24.88% |
| lactose | 49.76% |
| hypromellose-E5 | 2.93% |
| croscarmellose sodium | 9.76% |
| magnesium stearate | 0.49% |
| film coating premix | 2.44% |

The compound of formula (II) was passed through a 20-mesh sieve, and then the sieved active pharmaceutical ingredient was added into a jet mill and ground under the grinding pressure of 4 to 6 bar, the inlet pressure of 6 to 7 bar, the feed pressure of 5 to 6 bar, and the feed rotary speed of 200 to 300 rpm. Samples were taken and tested, and the particle size distribution was measured by a laser particle size distribution analyzer, showing the particle size D90 ≤ 20µm (D90 = 17.89 µm). The compound of formula (II) with D90 ≤ 20 µm (D90 = 17.89 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted and coated with a stomach-soluble film coating premix to prepare tablets having a specification of 410mg.

### Preparation Example 2-3 Coated tablet formulation

Tablet formulation comprising the compound of formula (II) (mass percent):

| | |
|---|---|
| compound of formula (II) | 9.76% |
| microcrystalline cellulose | 24.88% |
| lactose | 49.76% |
| hypromellose-E5 | 2.93% |
| croscarmellose sodium | 9.76% |
| magnesium stearate | 0.49% |
| film coating premix | 2.44% |

The compound of formula (II) was passed through a 20-mesh sieve, and then the sieved active pharmaceutical ingredient was added into a jet mill and ground under the grinding pressure of 6 to 7 bar, the inlet pressure of 7 to 8 bar, the feed pressure of 6 to 7 bar, and the feed rotary speed of 100 to 150 rpm. After the grinding was completed, samples were taken and tested, and the particle size distribution was measured by a laser particle size distribution analyzer, showing the particle size D90 ≤ 10µm (D90 = 7.461 µm).The compound of formula (II) with the particle size of D90 ≤ 10 µm (D90 = 7.461 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted and coated with a stomach-soluble film coating premix to prepare tablets having a specification of 205 mg.

### Preparation Example 2-4 Coated tablet formulation as reference example

Tablet formulation comprising the compound of formula (II) (mass percent):

| | |
|---|---|
| compound of formula (II) | 9.76% |
| microcrystalline cellulose | 24.88% |
| lactose | 49.76% |
| hypromellose-E5 | 2.93% |
| croscarmellose sodium | 9.76% |
| magnesium stearate | 0.49% |
| film coating premix | 2.44% |

The compound of formula (II) was passed through a 20-mesh sieve, and ground by a laboratory shear pulverizer, grinding 3 minutes with an interval of 5 minutes. After grinding for 10 minutes, the particle size distribution was measured by a laser particle size distribution analyzer, showing the particle size D90 ≤ 50µm (D90 = 45.71 µm). The compound of formula (II) with the particle size of D90 ≤ 50 µm (D90 = 45.71 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted and coated with a stomach-soluble film coating premix to prepare tablets having a specification of 205 mg.

### Preparation Example 2-5 Coated tablet formulation as reference example

Tablet formulation comprising the compound of formula (II) (mass percent):

| | |
|---|---|
| compound of formula (II) | 9.76% |
| microcrystalline cellulose | 24.88% |
| lactose | 49.76% |
| hypromellose-E5 | 2.93% |
| croscarmellose sodium | 9.76% |
| magnesium stearate | 0.49% |
| film coating premix | 2.44% |

After the compound of formula (II) was passed through a 20-mesh sieve, the particle size distribution was measured by a laser particle size distribution analyzer, showing the particle size D90 ≤ 150µm (D90 = 144.5 µm). The compound of formula (II) with the particle size of D90 ≤ 150 µm (D90 = 144.5 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted and coated with a stomach-soluble film coating premix to prepare tablets having a specification of 205 mg.

### Preparation Example 2-6 Uncoated plain tablet formulation

Tablet formulation comprising the compound of formula (II) (mass percent):

| | |
|---|---|
| compound of formula (II) | 10% |
| microcrystalline cellulose | 25.5% |
| lactose | 51% |
| hypromellose-E5 | 3% |
| croscarmellose sodium | 10% |
| magnesium stearate | 0.5% |

The compound of formula (II) was passed through a 20-mesh sieve, and then the sieved active pharmaceutical ingredient was added into a jet mill and ground under the grinding pressure of 4 to 6 bar, the inlet pressure of 6 to 7 bar, the feed pressure of 5 to 6 bar, and the feed rotary speed of 200 to 300 rpm. Samples were taken and tested, and the particle size distribution was measured by a laser particle size distribution analyzer, showing the particle size D90 ≤ 20µm (D90 = 17.89 µm). The compound of formula (II) with the particle size of D90 ≤ 20 µm (D90 = 17.89 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted to prepare uncoated tablets having a specification of 200mg.

### Preparation Example 2-7 Uncoated plain tablet formulation

Tablet formulation comprising the compound of formula (II) (mass percent):

| | |
|---|---|
| compound of formula (II) | 10% |
| microcrystalline cellulose | 25.5% |
| lactose | 51% |
| hypromellose-E5 | 3% |
| croscarmellose sodium | 10% |
| magnesium stearate | 0.5% |

The compound of formula (II) was passed through a 20-mesh sieve, and then the sieved active pharmaceutical ingredient was added into a jet mill and ground under the grinding pressure of 4 to 6 bar, the inlet pressure of 6 to 7 bar, the feed pressure of 5 to 6 bar, and the feed rotary speed of 200 to 300 rpm. Samples were taken and tested, and the particle size distribution was measured by a laser particle size distribution analyzer, showing the particle size D90 ≤ 20µm (D90 = 17.89 µm). The compound of formula (II) with the particle size of D90 ≤ 20 µm (D90 = 17.89 µm) and lactose were added, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted to prepare uncoated plain tablets having a specification of 400 mg.

### Preparation Example 2-8 Coated tablet formulation

Tablet formulation comprising the compound of formula (II) (mass percent):

| | |
|---|---|
| compound of formula (II) | 9.76% |
| microcrystalline cellulose | 24.88% |
| lactose | 49.76% |
| hypromellose-E5 | 2.93% |
| croscarmellose sodium | 9.76% |
| magnesium stearate | 0.49% |
| film coating premix | 2.44% |

The compound of formula (II) was passed through a 20-mesh sieve, and then the sieved active pharmaceutical ingredient was added into a jet mill and ground under the grinding pressure of 6 to 7 bar, the inlet pressure of 7 to 8 bar, the feed pressure of 6 to 7 bar, and the feed rotary speed of 100 to 150 rpm. After the grinding was completed, samples were taken and tested, and the particle size distribution was measured by a laser particle size distribution analyzer, showing the particle size D90 ≤ 10µm (D90 = 8.93 µm). The compound of formula (II) with the particle size of D90 ≤ 10 µm (D90 = 8.93 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted and coated with a stomach-soluble film coating premix to prepare tablets having a specification of 205 mg.

### Preparation Example 3 Uncoated plain tablet formulation

Tablet formulation comprising the compound of formula (II) (mass percent):

| | |
|---|---|
| compound of formula (II) | 4% |
| microcrystalline cellulose | 26.2% |
| lactose | 56.3% |
| hypromellose-E5 | 3% |
| croscarmellose sodium | 10% |
| magnesium stearate | 0.48% |

The compound of formula (II) was passed through a 20-mesh sieve, and then the sieved active pharmaceutical ingredient was added into a jet mill and ground under the grinding pressure of 6 to 7 bar, the inlet pressure of 7 to 8 bar, the feed pressure of 6 to 7 bar, and the feed rotary speed of 100 to 150 rpm. After the grinding was completed, samples were taken and tested, and the particle size distribution was measured by a laser particle size distribution analyzer, showing the particle size D90 ≤ 10µm (D90 = 7.461 µm).The compound of formula (II) with the particle size of D90 ≤ 10 µm (D90 = 7.461 µm), lactose, and microcrystalline cellulose were added into a wet mixing granulator, stirred and mixed. Then 10% (mass percent) hypromellose-E5 aqueous solution was slowly added for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer. The total moisture content of the granules after final mixing was ≤ 3.0%. The granules were tableted to prepare tablets having a specification of 500mg.

### Preparation Example 4-1 Uncoated plain tablet formulation

Tablet formulation comprising the compound of formula (II) (mass percent):

| | |
|---|---|
| compound of formula (II) | 10% |
| microcrystalline cellulose | 24.3% |
| lactose | 48.7% |
| hypromellose-E5 | 1.5% |
| hypromellose-K4M | 15% |
| magnesium stearate | 0.5% |

The compound of formula (II) was passed through a 20-mesh sieve, and then the sieved active pharmaceutical ingredient was added into a jet mill and ground under the grinding pressure of 4 to 6 bar, the inlet pressure of 6 to 7 bar, the feed pressure of 5 to 6 bar, and the feed rotary speed of 200 to 300 rpm. Samples were taken and tested, and the particle size distribution was measured by a laser particle size distribution analyzer, showing the particle size D90 ≤ 20µm (D90 = 17.89 µm).The compound of formula (II) with D90 ≤ 20 µm (D90 = 17.89 µm), lactose, microcrystalline cellulose and hypromellose-K4M were added into a wet mixing granulator, stirred and mixed. Then 10% (mass percent) hypromellose-E5 aqueous solution was slowly added for performing wet granulation. The resultant was subjected to wet granule sizing and dried in an oven. The moisture content of the dried granules was ≤ 2.0%. Then dry granule sizing was performed. The resulting granules after dry granule sizing, hypromellose-K4M and magnesium stearate were finally mixed in a mixer. The total moisture content of the granules after final mixing was ≤ 3.0%. The granules were tableted to prepare tablets having a specification of 200mg.

### Preparation Example 4-2 Uncoated plain tablet formulation

Tablet formulation comprising the compound of formula (II) (mass percent):

| | |
|---|---|
| compound of formula (II) | 10% |
| microcrystalline cellulose | 27.5% |
| lactose | 55% |
| hypromellose-E5 | 2% |
| hypromellose-K4M | 5% |
| magnesium stearate | 0.5% |

The compound of formula (II) was passed through a 20-mesh sieve, and then the sieved active pharmaceutical ingredient was added into a jet mill and ground under the grinding pressure of 4 to 6 bar, the inlet pressure of 6 to 7 bar, the feed pressure of 5 to 6 bar, and the feed rotary speed of 200 to 300 rpm. Samples were taken and tested, and the particle size distribution was measured by a laser particle size distribution analyzer, showing the particle size D90 ≤ 20µm (D90 = 17.89 µm).The compound of formula (II) with D90 ≤ 20 µm (D90 = 17.89 µm), lactose, microcrystalline cellulose and hypromellose-K4M were added into a wet mixing granulator, stirred and mixed. Then 10% (mass percent) hypromellose-E5 aqueous solution was slowly added for performing wet granulation. The resultant was subjected to wet granule sizing and dried in an oven. The moisture content of the dried granules was ≤ 2.0%. Then dry granule sizing was performed, and the resulting granules after dry granule sizing, hypromellose-K4M and magnesium stearate were finally mixed in a mixer. The total moisture content of the granules after final mixing was ≤ 3.0%. The granules were tableted to prepare tablets having a specification of 200mg.

### Preparation Example 4-3 Uncoated plain tablet formulation

Tablet formulation comprising the compound of formula (II) (mass percent):

| | |
|---|---|
| compound of formula (II) | 10% |
| microcrystalline cellulose | 25.8% |
| lactose | 51.7% |
| hypromellose-E5 | 2% |
| hypromellose-K4M | 10% |
| magnesium stearate | 0.5% |

The compound of formula (II) was passed through a 20-mesh sieve, and then the sieved active pharmaceutical ingredient was added into a jet mill and ground under the grinding pressure of 4 to 6 bar, the inlet pressure of 6 to 7 bar, the feed pressure of 5 to 6 bar, and the feed rotary speed of 200 to 300 rpm. Samples were taken and tested, and the particle size distribution was measured by a laser particle size distribution analyzer, showing the particle size D90 ≤ 20µm (D90 = 17.89 µm).The compound of formula (II) with D90 ≤ 20 µm (D90 = 17.89 µm), lactose, microcrystalline cellulose and hypromellose-K4M were added into a wet mixing granulator, stirred and mixed. Then 10% (mass percent) hypromellose-E5 aqueous solution was slowly added for performing wet granulation. The resultant was subjected to wet granule sizing and dried in an oven. The moisture content of the dried granules was ≤ 2.0%. Then dry granule sizing was performed, and the resulting granules after dry granule sizing, hypromellose-K4M and magnesium stearate were finally mixed in a mixer. The total moisture content of the granules after final mixing was ≤ 3.0%. The granules were tableted to prepare tablets having a specification of 200mg.

### Preparation Example 5 Coated tablet formulation

Tablet formulation comprising the compound of formula (II) (mass percent):

| | |
|---|---|
| compound of formula (II) | 9.8% |
| microcrystalline cellulose | 25% |
| lactose | 50% |
| hypromellose-E5 | 2.94% |
| croscarmellose sodium | 9.8% |
| magnesium stearate | 0.49% |
| film coating premix | 1.96% |

The compound of formula (II) was passed through a 20-mesh sieve, and then the sieved active pharmaceutical ingredient was added into a jet mill and ground under the grinding pressure of 4 to 6 bar, the inlet pressure of 6 to 7 bar, the feed pressure of 5 to 6 bar, and the feed rotary speed of 200 to 300 rpm. Samples were taken and tested, and the particle size distribution was measured by a laser particle size distribution analyzer, showing the particle size D90 ≤ 20µm (D90 = 17.89 µm).The compound of formula (II) with D90 ≤ 20 µm (D90 = 17.89 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting dry granules, croscarmellose sodium and magnesium stearate were finally mixed in a mixer. The total moisture content of the granules after final mixing was ≤ 3.0%. The granules were tableted and coated with a stomach-soluble film coating premix to prepare tablets having a specification of 102mg.

### Preparation Example 6 Coated tablet formulation

Tablet formulation comprising the compound of formula (II) (mass percent):

| | |
|---|---|
| compound of formula (II) | 9.76% |
| microcrystalline cellulose | 24.88% |
| lactose | 49.76% |
| hypromellose-E5 | 2.93% |
| croscarmellose sodium | 9.76% |
| magnesium stearate | 0.49% |
| film coating premix | 2.44% |

The compound of formula (II) (in crystalline form B) was passed through a 20-mesh sieve, and then the sieved active pharmaceutical ingredient was added into a jet mill and ground under the grinding pressure of 6 to 7 bar, the inlet pressure of 7 to 8 bar, the feed pressure of 6 to 7 bar, and the feed rotary speed of 100 to 150 rpm. Samples was taken and tested, and the particle size distribution was measured by a laser particle size distribution analyzer, showing the particle size D90 ≤ 20µm (D90 = 10.09 µm).The compound of formula (II) with the particle size of D90 ≤ 20 µm (D90 = 10.09 µm) and lactose were added into a wet mixing granulator, stirred and mixed, and then microcrystalline cellulose and croscarmellose sodium were added, stirred and mixed. Then 10% (mass percent) hypromellose-E5 aqueous solution was slowly added into the wet mixing granulator for performing wet granulation. The resultant was subjected to wet granule sizing and dried in an oven. The moisture content of the dried granules was ≤ 2.0%. Then dry granule sizing was performed. The resulting granules after dry granule sizing, croscarmellose sodium and magnesium stearate were finally mixed in a mixer, the total moisture content of the granules after final mixing was ≤ 3.0%. The granules were tableted and coated with a stomach-soluble film coating premix to prepare tablets having a specification of 205 mg.

### Test Example 1 In vitro dissolution test

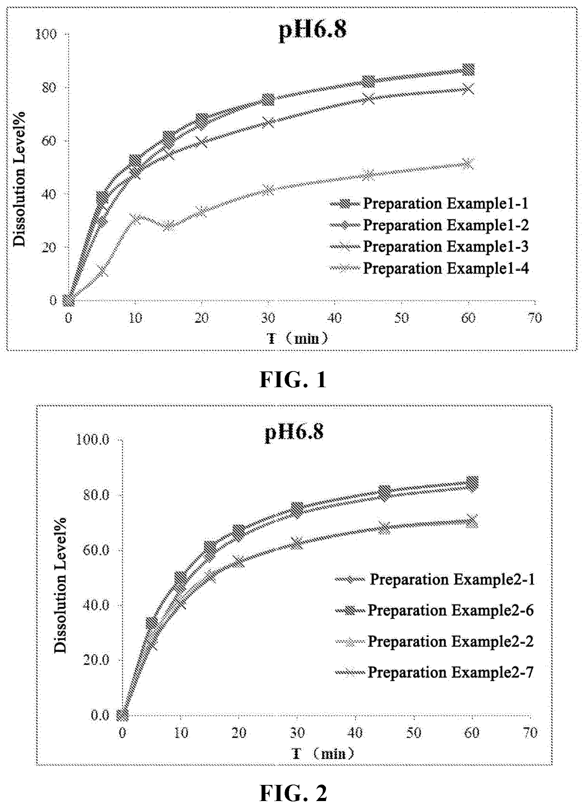

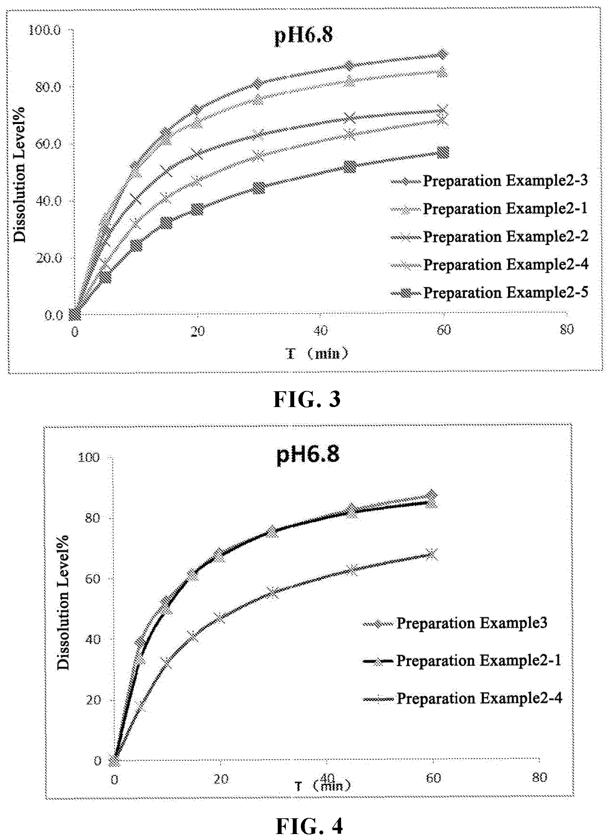

According to the second method of Chinese Pharmacopoeia 2015 4th Edition General Principles 0931, the tablet formulations prepared in the above preparation examples were subjected to an in vitro dissolution test to detect their dissolution levels in a phosphate buffer under pH 6.8, in which the dissolution device used paddle method, the dissolution medium was a pH 6.8 buffer, the water bath temperature was 37.0±0.5°C, the dissolution volume was 900ml, the rotation speed was 50 rpm, the sampling time was 5min, 10min, 15min, 20min, 30min, 45min, 60min, the sampling volume was 5ml, and the filter membrane was polyethersulfone filter. The test results were shown in the following Tables 1-1 to 1-5 and FIGs 1 to 5.

**Table 1-1 In vitro dissolution results of Preparation Examples 1-1 to 1-4**

| T, min | Preparation Example 1-4 | | Preparation Example 1-3 | | Preparation Example 1-2 | | Preparation Example 1-1 | |
|---|---|---|---|---|---|---|---|---|
| | Dissolution Level,% | RSD | Dissolution Level,% | RSD | Dissolution Level,% | RSD | Dissolution Level,% | RSD |
| 0 | 0 | 0 | 0 | 0 | 0.0 | 0 | 0 | 0 |
| 5 | 11 | 10.1 | 35.6 | 2.6 | 29.6 | 2.1 | 38.8 | 6.5 |
| 10 | 30.3 | 48.3 | 47.5 | 4.1 | 48.0 | 1.9 | 52.5 | 2.1 |
| 15 | 27.9 | 1.2 | 54.6 | 4.5 | 58.6 | 1.0 | 61.5 | 1.8 |
| 20 | 33.3 | 3.1 | 59.3 | 0.1 | 65.9 | 0.4 | 68.2 | 0.7 |
| 30 | 41.4 | 1.3 | 66.8 | 1.0 | 75.4 | 1.2 | 75.4 | 0.6 |
| 45 | 47.1 | 0.9 | 75.7 | 0.9 | 82.0 | 0.4 | 82.4 | 0.6 |
| 60 | 51.3 | 3.9 | 79.5 | 0.7 | 86.5 | 1.0 | 86.9 | 0.8 |

**Table 1-2 In vitro dissolution results of Preparation Examples 2-1, 2-2, 2-6 and 2-7**

| T, min | Preparation Example2-6 | | Preparation Example2-1 | | Preparation Example2-7 | | Preparation Example2-2 | |
|---|---|---|---|---|---|---|---|---|
| | Dissolution Level,% | RSD | Dissolution Level,% | RSD | Dissolution Level,% | RSD | Dissolution Level, % | RSD |
| 0 | 0 | 0 | 0 | 0 | 0.0 | 0 | 0 | 0 |
| 5 | 33.5 | 2.8 | 26.8 | 11.0 | 25.8 | 6.4 | 28.6 | 8.5 |
| 10 | 50.1 | 4.6 | 45.8 | 6.4 | 40.4 | 3.1 | 42.4 | 4.7 |
| 15 | 61.3 | 1.6 | 57.8 | 5.7 | 50.0 | 1.9 | 51.1 | 2.9 |
| 20 | 67.3 | 1.9 | 64.9 | 3.9 | 55.9 | 1.3 | 56.0 | 2.0 |
| 30 | 75.3 | 1.3 | 73.3 | 2.0 | 62.6 | 0.8 | 62.6 | 0.9 |
| 45 | 81.5 | 0.5 | 79.5 | 1.5 | 68.3 | 0.3 | 68.1 | 1.1 |
| 60 | 84.8 | 0.7 | 83.0 | 1.4 | 71.0 | 0.5 | 70.3 | 1.0 |

**Table 1-3 In vitro dissolution results of Preparation Examples 2-1 to 2-5**

| T, min | Preparation Example2-1 | | Preparation Example2-2 | | Preparation Example2-3 | | Preparation Example2-5 | | Preparation Example2-4 | |
|---|---|---|---|---|---|---|---|---|---|---|
| | Dissolution Level,% | RSD | Dissolution Level,% | RSD | Dissolution Level,% | RSD | Dissolution Level,% | RSD | Dissolution Level,% RSD | |
| 0 | 0 | 0 | 0 | 0 | 0.0 | 0 | 0 | 0 | 0 | 0 |
| 5 | 33.5 | 2.8 | 25.8 | 6.4 | 29.8 | 13.3 | 13.1 | 12.2 | 17.9 | 9.3 |
| 10 | 50.1 | 4.6 | 40.4 | 3.1 | 51.8 | 4.5 | 24.3 | 6.8 | 31.9 | 3.1 |
| 15 | 61.3 | 1.6 | 50.0 | 1.9 | 63.7 | 3.3 | 32.0 | 3.3 | 40.8 | 1.5 |
| 20 | 67.3 | 1.9 | 55.9 | 1.3 | 71.5 | 2.6 | 36.7 | 2.8 | 46.7 | 0.9 |
| 30 | 75.3 | 1.3 | 62.6 | 0.8 | 80.7 | 1.7 | 44.2 | 1.2 | 55.1 | 1.2 |
| 45 | 81.5 | 0.5 | 68.3 | 0.3 | 86.8 | 1.4 | 51.4 | 1.5 | 62.5 | 0.7 |
| 60 | 84.8 | 0.7 | 71.0 | 0.5 | 90.6 | 1.5 | 56.3 | 0.8 | 67.6 | 1.8 |

**Table 1-4 In vitro dissolution results of Preparation Examples 3, 2-1 and 2-4**

| T, min | Preparation Example3 | | Preparation Example2-1 | | Preparation Example2-4 | |
|---|---|---|---|---|---|---|
| | Dissolution Level,% | RSD | Dissolution Level,% | RSD | Dissolution Level,% | RSD |
| 0 | 0 | 0 | 0 | 0 | 0 | 0 |
| 5 | 38.8 | 6.5 | 33.5 | 2.8 | 17.9 | 9.3 |
| 10 | 52.5 | 2.1 | 50.1 | 4.6 | 31.9 | 3.1 |
| 15 | 61.5 | 1.8 | 61.3 | 1.6 | 40.8 | 1.5 |
| 20 | 68.2 | 0.7 | 67.3 | 1.9 | 46.7 | 0.9 |
| 30 | 75.4 | 0.6 | 75.3 | 1.3 | 55.1 | 1.2 |
| 45 | 82.4 | 0.6 | 81.5 | 0.5 | 62.5 | 0.7 |
| 60 | 86.9 | 0.8 | 84.8 | 0.7 | 67.6 | 1.8 |

**Table 1-5 In vitro dissolution results of Preparation Examples 4-1 to 4-3**

| T, h | Preparation Example4-1 | | Preparation Example4-2 | | Preparation Example4-3 | |
|---|---|---|---|---|---|---|
| | Dissolution Level,% | RSD | Dissolution Level,% | RSD | Dissolution Level,% | RSD |
| 0 | 0.0 | 0 | 0.0 | 0 | 0.0 | 0 |
| 0.25 | 3.7 | 46.2 | 25.3 | 15.6 | 10.3 | 27.5 |
| 0.5 | 7.5 | 29.2 | 45.6 | 11.6 | 18.0 | 30.7 |
| 1 | 12.4 | 23.9 | 67.4 | 6.9 | 27.9 | 23.4 |
| 2 | 20.5 | 18.2 | 84.2 | 3.7 | 42.8 | 17.2 |
| 4 | 32.0 | 14.2 | 96.0 | 2.5 | 70.9 | 6.5 |
| 6 | 41.4 | 9.8 | 97.9 | 2.6 | 88.1 | 2.1 |
| 8 | 49.8 | 8.8 | 98.9 | 2.6 | 96.5 | 1.6 |
| 24 | 93.8 | 3.2 | 101.9 | 2.4 | 102.1 | 1.9 |

**Table 1-6 In vitro dissolution results of Preparation Examples 2-8 and 6**

| ,T, min | pH6.8 | | | | 0.1N HCL | | | |
|---|---|---|---|---|---|---|---|---|
| | **Preparation Example2-8** | | **Preparation Example6** | | **Preparation Example2-8** | | **Preparation Example6** | |
| | **Dissolution Level,%** | RSD, % | **Dissolution Level,%** | RSD, % | **Dissolution Level,%** | RSD, % | **Dissolution Level,%** | RSD ,% |
| 00 | 0 | 0 | 0.0 | 0 | 0 | 0 | 0.0 | 0 |
| 55 | 41.9 | 7.3 | 59.3 | 6.6 | 59.8 | 12.6 | 65.9 | 14.2 |
| 110 | 62.3 | 10.8 | 74.6 | 3.0 | 73.6 | 6.3 | 82.1 | 8.2 |
| 115 | 67.7 | 2.0 | 81.3 | 2.6 | 79.8 | 7.5 | 89.5 | 5.6 |
| 220 | 71.1 | 1.9 | 85.0 | 2.0 | 82.6 | 5.1 | 94.1 | 2.1 |
| 330 | 75.6 | 1.2 | 91.1 | 2.2 | 87.8 | 4.2 | 99.5 | 1.5 |
| 445 | 80.1 | 1.3 | 96.4 | 2.0 | 92.7 | 2.5 | 102.7 | 1.2 |
| 660 | 83.0 | 2.2 | 99.3 | 1.6 | 94.4 | 0.6 | 103.8 | 1.2 |

### Test Example 2 Stability Test

After placing the prepared samples under high temperature of 60°C and accelerated conditions (40°C/75%RH) for a certain period of time, the samples were tested for content, related substances and dissolution level according to the Second method of Chinese Pharmacopoeia 2015 4th Edition General Principles 0512 and 0931, to assess the stability thereof. The test results were shown in Table 2-1.

**Table 2-1 Stability results of Preparation Examples 2-1, 2-2 and 2-4**

| **Preparation Example** | **Conditions** | **Single Impurity,%** | | **Total Impurity, %** | **Content, %** | **Dissolution Level,%** |
|---|---|---|---|---|---|---|
| | | **Unknown Impurity** | **Other Impurities** | | | |
| | | RRT1.08 | RRT1.25 | | | |
| | | ≤0.30% | ≤0.25% | ≤1.5% | | |
| **Preparation Example2-1** | 0d | 0.07 | 0.14 | 0.21 | 97.7 | 84.3 |
| | 60°C,1M | 0.07 | 0.14 | 0.21 | 98.9 | / |
| | 40°C/75%RH,1M | 0.07 | 0.14 | 0.21 | 98.3 | / |
| | 60°C,2M | 0.04 | 0.12 | 0.16 | 98.3 | / |
| | 40°C/75%RH,2M | 0.04 | 0.13 | 0.17 | 98.9 | / |
| **Preparation Example2-4** | 0d | 0.05 | 0.12 | 0.17 | 100.9 | / |
| | 60°C,1M | 0.06 | 0.12 | 0.18 | 98.7 | / |
| | 40°C/75%RH,1M | 0.06 | 0.12 | 0.18 | 97.4 | / |
| **Preparation Example2-2** | 0d | 0.05 | 0.16 | 0.21 | 95.6 | / |
| | 60°C,1M | 0.05 | 0.15 | 0.20 | 96.1 | / |
| | 40°C/75%RH, 1M | 0.05 | 0.16 | 0.21 | 97.2 | / |
| | 60°C,2M | 0.04 | 0.13 | 0.17 | 104.1 | / |
| | 40°C /75%RH,2M | 0.04 | 0.13 | 0.17 | 104.3 | / |
| | 40°C/75%RH,2M | 0.06 | 0.13 | 0.19 | 97.7 | 87.5 |

### Test Example 3 Dog oral bioavailability test

A dog oral bioavailability research was performed on the prepared samples. The research was designed according to the technical guidelines for non-clinical pharmacokinetic research of the former China Food and Drug Administration (CFDA) and ICH M3(R2). The tablet has a specification of 20 mg, 1 tablet/1 dog. The samples were collected according to the designed time point. The concentrations of the active ingredients in the samples were detected by HPLC-MS/MS method, and the pharmacokinetic parameters were calculated. The specific test contents were as follows:
Animals and administration: common grade 7-14 month old Beagle dogs weight of 9.51kg-11.14kg were used. Oral administration was performed with single administration on each administration day with administration time 8:00-12:00. All animals were fasted overnight before each administration, and continued to fast for 2-3 hours after administration, but the total fasting time did not exceed 24 hours.

Blood collection: forelimb venous blood collection was used. Blood collection time was each administration day (i.e., Day 1, Day 5, Day 8, Day 12 and Day 15). Blood collection time points were before administration (0h), 5min, 15min, 30min, 1h, 2h, 4h, 6h, 8h and 24h after administration. About 1 mL of blood was collected in EDTA dipotassium anticoagulant vacuum blood collection tube. After blood collection, the blood collection tube was shaken gently to mix blood and anticoagulant thoroughly. Blood was placed in crushed ice after collection, and centrifuged (4°C, 2000 g, 10 min) within 1 hour after collection. After centrifugation, about 400 µL of plasma was collected in a brown plastic tube, and immediately stored in a container of dry ice, then transferred to an ultra-low temperature refrigerator (≤-65°C) and stored in the dark.

Sample analysis: HPLC-MS/MS method was used to detect the concentration of the active ingredients in the sample, and Microsoft Excel 2013 was used for data processing and calculation. After analyzing and measuring the concentration of samples in plasma, the concentration-time curve was plotted by WinNonlin6.3 software, and the pharmacokinetic parameters were calculated according to the non-compartmental model. The results of the pharmacokinetic parameters of the corresponding formulations are shown in the following Table 3-1, Table 3-2 and Table 3-3, respectively. The results in the same table are obtained by testing in the same batch, and the results in different tables are obtained by testing in different batches.

**Table 3-1 Oral bioavailability of Preparation Examples 1-4, 1-3 and 1-2**

| **Preparation Example** | **Preparation Example1-4** | **Preparation Example1-3** | **Preparation Example1-2** |
|---|---|---|---|
| | | 2mpk | |
| AUC₀₋ₜ (hr*ng/mL) | 888 | 1537 | 2096 |
| AUC_{0-∞} (hr*ng/mL) | 1069 | 1804 | 2169 |
| Tₘₐₓ(h) | 1.17 | 0.667 | 1.33 |
| Cₘₐₓ(ng/mL) | 288 | 708 | 827 |
| F(%) | 53.1 | 89.7 | 108 |

**Table 3-2 Oral bioavailability of Preparation Examples 2-1, 2-3 and 2-5**

| **Preparation Example** | **Preparation Example2-3** | **Preparation Example2-1** | **Preparation Example2-5** |
|---|---|---|---|
| Tₘₐₓ (h) | 1.13 ± 0.737 | 1.08 ± 0.492 | 2.25 ± 2.86 |
| Cₘₐₓ (ng/mL) | 383 ± 372 | 533 ± 520 | 143 ± 103 |
| AUC₀₋ₜ (hr*ng/mL) | 1205 ± 1032 | 1252 ± 990 | 722 ± 812 |
| AUC_{0-∞} (hr*ng/mL) | 1297 ± 1121 | 1272 ± 976 | 883 ± 1022 |

**Table 3-3 Oral bioavailability of Preparation Examples 3, 2-1, 2-3 and 2-4**

| **Preparation Example** | T_{1/2} (h) | Tₘₐₓ (h) | Cₘₐₓ (ng/mL) | AUC₀₋ₜ (ng*h/mL) |
|---|---|---|---|---|
| **Preparation Example3** | 1.38 | 0.875 | 436 | 1000 |
| **Preparation Example2-1** | 1.88 | 1.25 | 708 | 1060 |
| **Preparation Example2-4** | 6.17 | 0.875 | 393 | 901 |
| **Preparation Example2-3** | 0.79 | 1.25 | 613 | 927 |

It can be seen from the table above that different particle sizes of the active ingredient in the formulation have different effects on pharmacokinetic parameters, such as prolonged half-life or decreased exposure in Beagle dogs.

## Claims

1. A solid pharmaceutical formulation, wherein the solid pharmaceutical formulation comprises an active ingredient, and the active ingredient is a compound represented by formula (II) or a pharmaceutically acceptable salt thereof, or a mixture of both; 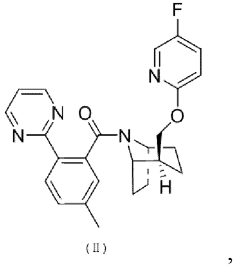
the particle size of the active ingredient is D90 ≤ 35 µm,
wherein the particle size of the active ingredient is measured by a laser particle size distribution analyzer,
wherein the content of the active ingredient is 1%-15% based on the total dry weight of the solid pharmaceutical formulation;
wherein the solid pharmaceutical formulation further comprises a binder selected from the group consisting of hypromellose, hydroxypropyl cellulose, povidone, sodium alginate, carbopol, polyvinyl alcohol and a combination thereof, wherein the content of the binder is 0.5%-10% based on the total dry weight of the solid pharmaceutical formulation;
wherein the solid pharmaceutical formulation further comprises a filler selected from the group consisting of microcrystalline cellulose, lactose, cellulose-lactose complex, pre-gelatinized starch, calcium hydrogen phosphate, calcium carbonate and a combination thereof, wherein the content of the filler is 60%-90% based on the total dry weight of the solid pharmaceutical formulation;
wherein the solid pharmaceutical formulation further comprises a disintegrant selected from the group consisting of croscarmellose sodium, hypromellose-K4M, crospovidone, sodium carboxymethyl starch and a combination thereof, wherein the content of the disintegrant is 5%-15% based on the total dry weight of the solid pharmaceutical formulation; and
wherein the solid pharmaceutical formulation further comprises a lubricant selected from the group consisting of magnesium stearate, talcum powder, glycerol monostearate, sodium stearyl fumarate and a combination thereof, wherein the content of the lubricant is 0.1%-1% based on the total dry weight of the solid pharmaceutical formulation.

2. The solid pharmaceutical formulation according to claim 1, wherein the particle size of the active ingredient is D90 ≤ 20 µm.

3. The solid pharmaceutical formulation according to claim 1, wherein the solid pharmaceutical formulation is a tablet, a capsule, a powder, a granule, a drop pill or a film, preferably a tablet.

4. The solid pharmaceutical formulation according to claim 1, wherein the solid pharmaceutical formulation comprises the following components based on the total dry weight of the solid pharmaceutical formulation:
a) the active ingredient: ((1S,2R,5S)-2-(((5-fluoropyridin-2-yl)oxy)methyl)-8-azabicyclo[3.2.1]octan-8-yl)(5-methyl-2-(pyrimidin-2-yl)phenyl)methanone, or a pharmaceutically acceptable salt thereof, or a mixture of both, wherein the content of the active ingredient is 4%-10%, more preferably 9.5%-10%;
b) a filler selected from the group consisting of microcrystalline cellulose, lactose, cellulose-lactose complex, pre-gelatinized starch, calcium hydrogen phosphate, calcium carbonate and a combination thereof, wherein the content of the filler is 73%-85%, more preferably 73%-82.5%;
c) a binder selected from the group consisting of hypromellose, hydroxypropyl cellulose, povidone, sodium alginate, carbopol, polyvinyl alcohol and a combination thereof, wherein the content of the binder is 1.5%-3%;
d) a disintegrant selected from the group consisting of croscarmellose sodium, hypromellose-K4M, crospovidone, sodium carboxymethyl starch and a combination thereof, wherein the content of the disintegrant is 5%-15%; and
e) a lubricant selected from the group consisting of magnesium stearate, talcum powder, glycerol monostearate, sodium stearyl fumarate and a combination thereof, wherein the content of the lubricant is 0.4%-0.5%, more preferably 0.48%-0.5%;
wherein the particle size of the active ingredient is D90 ≤ 35 µm, or D90 ≤ 30 µm, or D90 ≤ 20 µm, or D90 ≤ 10 µm, or D90 = 1 µm to 30 µm, or D90 = 1 µm to 20 µm, or D90 = 1 µm to 10 µm, or D90 = 10 µm to 20 µm.

5. The solid pharmaceutical formulation according to claim 1, wherein the solid pharmaceutical formulation comprises the following components based on the total dry weight of the solid pharmaceutical formulation:
a) the active ingredient: ((1S,2R,5S)-2-(((5-fluoropyridin-2-yl)oxy)methyl)-8-azabicyclo[3.2.1]octan-8-yl)(5-methyl-2-(pyrimidin-2-yl)phenyl)methanone, or a pharmaceutically acceptable salt thereof, or a mixture of both, wherein the content of the active ingredient is 4%-10%;
b) microcrystalline cellulose with a content of 24%-27.5%;
c) lactose with a content of 48.5%-56.5%;
d) hypromellose-E5 with a content of 1.5%-3%;
e) croscarmellose sodium or hypromellose-K4M with a content of 5%-15%; and
f) magnesium stearate with a content of 0.4%-0.5%;
wherein the particle size of the active ingredient is D90 ≤ 35 µm, or D90 ≤ 30 µm, or D90 ≤ 20 µm, or D90 ≤ 10 µm, or D90 = 1 µm to 30 µm, or D90 = 1 µm to 20 µm, or D90 = 1 µm to 10 µm, or D90 = 10 µm to 20 µm.

6. The solid pharmaceutical formulation according to claim 1, wherein the solid pharmaceutical formulation comprises the following components based on the total dry weight of the solid pharmaceutical formulation:
a) the active ingredient: ((1S,2R,5S)-2-(((5-fluoropyridin-2-yl)oxy)methyl)-8-azabicyclo[3.2.1]octan-8-yl)(5-methyl-2-(pyrimidin-2-yl)phenyl)methanone, or a pharmaceutically acceptable salt thereof, or a mixture of both, wherein the content of the active ingredient is 9.5%-10%;
b) microcrystalline cellulose with a content of 24%-27.5%;
c) lactose with a content of 48.5%-56.5%;
d) hypromellose-E5 with a content of 1.5%-3%;
e) croscarmellose sodium or hypromellose-K4M with a content of 5%-15%; and
f) magnesium stearate with a content of 0.48%-0.5%;
wherein the particle size of the active ingredient is D90 = 1 µm to 30 µm, or D90 = 1 µm to 20 µm, or D90 = 1 µm to 10 µm, or D90 = 10 µm to 20 µm, and the solid pharmaceutical formulation is a tablet.

7. A method for preparing the solid pharmaceutical formulation according to claim 3, wherein the solid pharmaceutical formulation is a tablet, wherein the method comprises the following steps:
(a) performing wet granulation after mixing an active ingredient particle, a filler, a binder and a first disintegrant;
(b) drying a resulting product obtained in step (a);
(c) dry-blending a resulting product obtained in step (b), a second disintegrant and a lubricant; and
(d) compressing a resulting product obtained in step (c) into the tablet;
wherein the active ingredient is ((1S,2R,5S)-2-(((5-fluoropyridin-2-yl)oxy)methyl)-8-azabicyclo[3.2.1]octan-8-yl)(5-methyl-2-(pyrimidin-2-yl)phenyl)methanone, or a pharmaceutically acceptable salt thereof, or a mixture of both; and
the particle size of the active ingredient particle is D90 ≤ 35µm.

8. The method according to claim 7, wherein the content of the active ingredient is 4%-10% based on the total dry weight of the mixture obtained in step (c).

9. The method according to claim 7, wherein the content of the filler is 73%-82.5% based on the total dry weight of the mixture obtained in step (c).

10. The method according to claim 7, wherein the content of the binder is 1.5%-3% based on the total dry weight of the mixture obtained in step (c).

11. The method according to claim 7, wherein the content of the first disintegrant is 2%-10%, more preferably 5%-6% based on the total dry weight of all components in step (a); and wherein the content of the second disintegrant is 5%-10% based on the total dry weight of all components in step (c).

12. The method according to claim 7, wherein the content of the lubricant is 0.1%-1%, more preferably 0.4%-0.5%, more preferably 0.48%-0.5% based on the total dry weight of all components in step (c).

13. The method according to claim 7, wherein the method further comprises: (e) coating a resulting product obtained in step (d).

14. A unit dosage form, wherein based on a total weight of the unit dosage form, the unit dosage form comprises:
10 mg, 20 mg, or 40 mg of an active ingredient, wherein the active ingredient is a compound of formula (II); 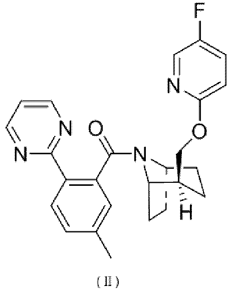
25 mg to 150 mg of microcrystalline cellulose;
50 mg to 300 mg of lactose;
1 mg to 15 mg of hypromellose;
10 mg to 50 mg of croscarmellose sodium; and
0.5 mg to 3 mg of magnesium stearate,
wherein the particle size of the active ingredient is D90 = 1 µm to 20 µm, and
wherein the particle size of the active ingredient is measured by a laser particle size distribution analyzer.

15. A unit dosage form, wherein based on a total weight of the unit dosage form, the unit dosage form comprises:
8 mg to 12 mg of an active ingredient, wherein the active ingredient is a compound of formula (II); 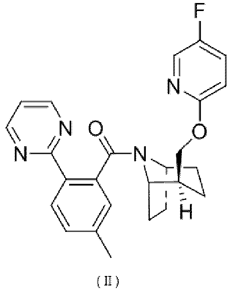
20 mg to 30 mg of microcrystalline cellulose;
46 mg to 56 mg of lactose;
2 mg to 4 mg of hypromellose;
5 mg to 15 mg of croscarmellose sodium; and
0.3 mg to 0.7 mg of magnesium stearate,
wherein the particle size of the active ingredient is D90 = 1 µm to 20 µm, and wherein the particle size of the active ingredient is measured by a laser particle size distribution analyzer.

16. A unit dosage form, wherein based on a total weight of the unit dosage form, the unit dosage form comprises:
18 mg to 22 mg of an active ingredient, wherein the active ingredient is a compound of formula (II); 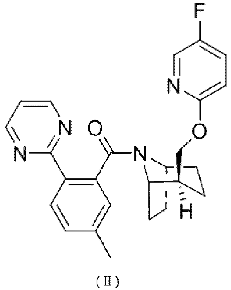
46 mg to 56 mg of microcrystalline cellulose;
97 mg to 107 mg of lactose;
5 mg to 7 mg of hypromellose;
15 mg to 25 mg of croscarmellose sodium; and
0.8 mg to 1.2 mg of magnesium stearate,
wherein the particle size of the active ingredient is D90 = 1 µm to 20 µm, and
wherein the particle size of the active ingredient is measured by a laser particle size distribution analyzer.

17. A unit dosage form, wherein based on a total weight of the unit dosage form, the unit dosage form comprises:
38 mg to 42 mg of an active ingredient, wherein the active ingredient is a compound of formula (II); 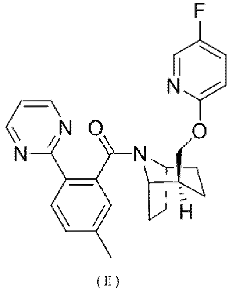
97 mg to 107 mg of microcrystalline cellulose;
199 mg to 209 mg of lactose;
11 mg to 13 mg of hypromellose;
35 mg to 45 mg of croscarmellose sodium; and
1.8 mg to 2.2 mg of magnesium stearate,
wherein the particle size of the active ingredient is D90 = 1 µm to 20 µm, and
wherein the particle size of the active ingredient is measured by a laser particle size distribution analyzer.

18. The solid pharmaceutical formulation according to any one of claims 1 to 6 or the unit dosage form according to any one of claims 14 to 17, wherein the compound of formula (II) as the active ingredient exists in crystalline form A or crystalline form B;
wherein the crystalline form A has Cu-Kα radiation XRPD spectrum:
| **Diffraction Angle 2θ** | **Relative Intensity (%)** | **Diffraction Angle 2θ** | **Relative Intensity (%)** |
|---|---|---|---|
| 10.428 | 60.0 | 25.533 | 8.2 |
| 11.968 | 100.0 | 26.083 | 11.0 |
| 13.542 | 17.8 | 27.133 | 3.1 |
| 14.238 | 3.4 | 27.506 | 17.7 |
| 14.767 | 12.1 | 28.316 | 3.0 |
| 15.851 | 73.9 | 29.557 | 2.2 |
| 16.818 | 72.0 | 30.740 | 4.2 |
| 18.003 | 9.2 | 31.846 | 5.1 |
| 19.087 | 2.1 | 32.550 | 2.0 |
| 19.755 | 47.6 | 33.775 | 3.1 |
| 20.900 | 25.7 | 34.682 | 2.2 |
| 22.652 | 2.9 | 36.422 | 3.4 |
| 23.858 | 49.2 | 36.953 | 3.4 |
| 24.844 | 23.2 | 38.569 | 3.0 |
; and wherein the crystalline form B has Cu-Kα radiation XRPD spectrum:
| **Diffraction Angle2θ** | **Relative Intensity (%)** | **Diffraction Angle2θ** | **Relative Intensity (%)** |
|---|---|---|---|
| 8.611 | 100.0 | 23.953 | 2.6 |
| 10.330 | 70.6 | 24.370 | 32.3 |
| 12.284 | 13.4 | 26.501 | 29.4 |
| 13.703 | 23.9 | 26.915 | 8.7 |
| 14.057 | 5.6 | 27.663 | 11.0 |
| 14.351 | 4.8 | 27.939 | 15.5 |
| 14.848 | 9.2 | 29.499 | 2.6 |
| 15.576 | 7.5 | 30.604 | 2.2 |
| 16.602 | 31.6 | 31.727 | 13.3 |
| 17.250 | 71.3 | 32.969 | 2.6 |
| 17.902 | 90.7 | 33.378 | 2.8 |
| 18.277 | 42.4 | 33.777 | 3.0 |
| 19.149 | 6.5 | 34.825 | 1.6 |
| 20.625 | 21.7 | 36.399 | 5.3 |
| 21.550 | 42.7 | 37.366 | 4.3 |
| 22.124 | 2.3 | 39.357 | 3.3 |
| 23.307 | 33.4 | | |

19. The unit dosage form according to any one of claims 14 to 17, wherein the unit dosage form is a tablet or a capsule.

20. The solid pharmaceutical formulation according to any one of claims 1 to 6, or the unit dosage form according to any one of claims 14 to 17, for use in treating an orexin-associated disease.

21. The solid pharmaceutical formulation or the unit dosage form for use according to claim 20, wherein the orexin-associated disease include insomnia, chronic obstructive pulmonary disease, obstructive sleep apnea, somnolence, anxiety, obsessive-compulsive disorder, panic, nicotine dependence or eating disorder.

## Patentansprüche

1. Feste pharmazeutische Formulierung, wobei die feste pharmazeutische Formulierung einen Wirkstoff umfasst, und der Wirkstoff eine Verbindung dargestellt durch die Formel (II) oder ein pharmazeutisch akzeptables Salz davon oder eine Mischung aus beiden ist; 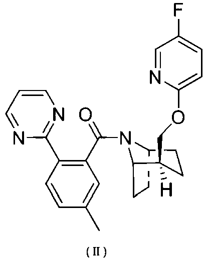
wobei die Partikelgröße des Wirkstoffs D90 ≤ 35 µm beträgt,
wobei die Partikelgröße des Wirkstoffs mit einem Laser-Partikelgrößenanalysator gemessen wird,
wobei der Gehalt des Wirkstoffs 1% bis 15% in Bezug auf das gesamte Trockengewicht der festen pharmazeutischen Formulierung beträgt;
wobei die feste pharmazeutische Formulierung ferner ein Bindemittel umfasst, das aus der Gruppe ausgewählt ist, die aus Hypromellose, Hydroxypropylcellulose, Povidon, Natriumalginat, Carbopol, Polyvinylalkohol und einer Kombination davon besteht, wobei der Gehalt des Bindemittels 0,5% bis 10% in Bezug auf das Gesamt-Trockengewicht der festen pharmazeutischen Formulierung beträgt;
wobei die feste pharmazeutische Formulierung ferner einen Füllstoff umfasst, der aus der Gruppe ausgewählt ist, die aus mikrokristalliner Cellulose, Lactose, Cellulose-Lactose-Komplex, vorgelatinierter Stärke, Calciumhydrogenphosphat, Calciumcarbonat und einer Kombination davon besteht, wobei der Gehalt des Füllstoffs 60% bis 90% beträgt, bezogen auf das Gesamt-Trockengewicht der festen pharmazeutischen Formulierung;
wobei die feste pharmazeutische Formulierung ferner ein Sprengmittel umfasst, das aus der Gruppe ausgewählt ist, die aus Croscarmellose-Natrium, Hypromellose-K4M, Crospovidon, Natriumcarboxymethylstärke und einer Kombination davon, wobei der Gehalt des Sprengmittels 5% bis 15% in Bezug auf das Gesamt-Trockengewicht der festen pharmazeutischen Formulierung beträgt; und wobei die feste pharmazeutische Formulierung ferner ein Gleitmittel umfasst, ausgewählt aus der Gruppe bestehend aus Magnesiumstearat, Talkumpuder, Glycerinmonostearat, Natriumstearylfumarat und einer Kombination davon, wobei der Gehalt des Gleitmittels 0,1% bis 1% in Bezug auf das Gesamt-Trockengewicht der festen pharmazeutischen Formulierung beträgt.

2. Feste pharmazeutische Formulierung nach Anspruch 1, wobei die Partikelgröße des Wirkstoffs D90 ≤ 20 µm beträgt.

3. Feste pharmazeutische Formulierung nach Anspruch 1, wobei die feste pharmazeutische Formulierung eine Tablette, eine Kapsel, ein Pulver, ein Granulat, eine Tropfenpille oder ein Überzug, vorzugsweise für eine Tablette, ist.

4. Feste pharmazeutische Formulierung nach Anspruch 1, wobei die feste pharmazeutische Formulierung die folgenden Komponenten, bezogen auf das Gesamt-Trockengewicht der festen pharmazeutischen Formulierung, umfasst:
a) den Wirkstoff: ((1S,2R,5S)-2-(((5-Fluorpyridin-2-yl)oxy)methyl)-8-azabicyclo[3.2.1]octan-8-yl)(5-methyl-2-(pyrimidin-2-yl)phenyl)methanon, oder ein pharmazeutisch akzeptables Salz davon oder eine Mischung aus beiden, wobei der Gehalt des Wirkstoffs 4% bis 10%, vorzugsweise 9,5% bis 10% beträgt;
b) ein Füllstoff, ausgewählt aus der Gruppe bestehend aus mikrokristalliner Cellulose, Lactose, Cellulose-Lactose-Komplex, vorgelatinierter Stärke, Calciumhydrogenphosphat, Calciumcarbonat und einer Kombination davon, wobei der Gehalt des Füllstoffs 73% bis 85%, vorzugsweise 73% bis 82,5% beträgt;
c) ein Bindemittel, ausgewählt aus der Gruppe bestehend aus Hypromellose, Hydroxypropylcellulose, Povidon, Natriumalginat, Carbopol, Polyvinylalkohol und einer Kombination davon, wobei der Gehalt des Bindemittels 1,5% bis 3% beträgt;
d) ein Sprengmittel, ausgewählt aus der Gruppe bestehend aus Croscarmellose-Natrium, Hypromellose-K4M, Crospovidon, Natriumcarboxymethylstärke und einer Kombination davon, wobei der Gehalt des Sprengmittels 5% bis 15% beträgt; und
e) ein Gleitmittel, ausgewählt aus der Gruppe bestehend aus Magnesiumstearat, Talkumpuder, Glycerinmonostearat, Natriumstearylfumarat und einer Kombination davon, wobei der Gehalt des Gleitmittels 0,4% bis 0,5%, vorzugsweise 0,48% bis 0,5%, beträgt;
wobei die Partikelgröße des Wirkstoffs D90 ≤ 35 µm, oder D90 ≤ 30 µm, oder D90 ≤ 20 µm, oder D90 ≤ 10 µm, oder D90 = 1 µm bis 30 µm, oder D90 = 1 µm bis 20 µm, oder D90 = 1 µm bis 10 µm, oder D90 = 10 µm bis 20 µm beträgt.

5. Feste pharmazeutische Formulierung gemäß Anspruch 1, wobei die feste pharmazeutische Formulierung die folgenden Komponenten, in Bezug auf das Gesamt-Trockengewicht der festen pharmazeutischen Formulierung, umfasst:
a) den Wirkstoff: ((1S,2R,5S)-2-(((5-Fluorpyridin-2-yl)oxy)methyl)-8-azabicyclo[3.2.1]octan-8-yl)(5-methyl-2-(pyrimidin-2-yl)phenyl)methanon, oder ein pharmazeutisch verträgliches Salz davon oder eine Mischung aus beiden, wobei der Gehalt des Wirkstoffs 4% bis 10% beträgt;
b) mikrokristalline Cellulose mit einem Gehalt von 24% bis 27,5%;
c) Lactose mit einem Gehalt von 48,5% bis 56,5%;
d) Hypromellose-E5 mit einem Gehalt von 1,5% bis 3%;
e) Croscarmellose-Natrium oder Hypromellose-K4M mit einem Gehalt von 5% bis 15%; und
f) Magnesiumstearat mit einem Gehalt von 0,4% bis 0,5%;
wobei die Partikelgröße des Wirkstoffs D90 ≤ 35 µm, oder D90 ≤ 30 µm, oder D90 ≤ 20 µm, oder D90 ≤ 10 µm, oder D90 = 1 µm bis 30 µm, oder D90 = 1 µm bis 20 µm, oder D90 = 1 µm bis 10 µm, oder D90 = 10 µm bis 20 µm beträgt.

6. Feste pharmazeutische Formulierung gemäß Anspruch 1, wobei die feste pharmazeutische Formulierung die folgenden Komponenten, in Bezug auf das Gesamt-Trockengewicht der festen pharmazeutischen Formulierung, umfasst:
a) den Wirkstoff: ((1S,2R,5S)-2-(((5-Fluorpyridin-2-yl)oxy)methyl)-8-azabicyclo[3.2.1]octan-8-yl)(5-methyl-2-(pyrimidin-2-yl)phenyl)methanon, oder ein pharmazeutisch verträgliches Salz davon oder eine Mischung aus beiden, wobei der Gehalt des Wirkstoffs 9,5% bis 10% beträgt;
b) mikrokristalline Cellulose mit einem Gehalt von 24% bis 27,5%;
c) Lactose mit einem Gehalt von 48,5% bis 56,5%;
d) Hypromellose-E5 mit einem Gehalt von 1,5% bis 3%;
e) Croscarmellose-Natrium oder Hypromellose-K4M mit einem Gehalt von 5% bis 15%; und
f) Magnesiumstearat mit einem Gehalt von 0,48% bis 0,5%;
wobei die Partikelgröße des Wirkstoffs D90 = 1 µm bis 30 µm oder D90 = 1 µm bis 20 µm oder D90 = 1 µm bis 10 µm oder D90 = 10 µm bis 20 µm beträgt und die feste pharmazeutische Formulierung eine Tablette ist.

7. Verfahren zur Herstellung der festen pharmazeutischen Formulierung gemäß Anspruch 3, wobei die feste pharmazeutische Formulierung eine Tablette ist, wobei das Verfahren die folgenden Schritte umfasst:
(a) Durchführen einer Nassgranulierung nach dem Mischen eines Wirkstoffpartikels, eines Füllstoffs, eines Bindemittels und eines ersten Sprengmittels;
(b) Trocknen eines in Schritt (a) erhaltenen Produkts;
(c) Trockenmischen eines in Schritt (b) erhaltenen Produkts, eines zweiten Sprengmittels und eines Gleitmittels; und
(d) Verpressen eines in Schritt (c) erhaltenen Produkts zu einer Tablette;
wobei der Wirkstoff ((1S,2R,5S)-2-(((5-Fluorpyridin-2-yl)oxy)methyl)-8-azabicyclo[3.2.1]octan-8-yl)(5-methyl-2-(pyrimidin-2-yl)phenyl)methanon oder ein pharmazeutisch verträgliches Salz davon oder eine Mischung aus beiden ist; und
die Partikelgröße des Wirkstoffpartikels D90 ≤ 35 µm beträgt.

8. Verfahren nach Anspruch 7, wobei der Gehalt des Wirkstoffs 4% bis 10% bezogen auf das Gesamt-Trockengewicht der in Schritt (c) erhaltenen Mischung beträgt.

9. Verfahren nach Anspruch 7, wobei der Gehalt des Füllstoffs 73% bis 82,5% bezogen auf das Gesamt-Trockengewicht der in Schritt (c) erhaltenen Mischung beträgt.

10. Verfahren nach Anspruch 7, wobei der Gehalt des Bindemittels 1,5% bis 3% bezogen auf das Gesamt-Trockengewicht der in Schritt (c) erhaltenen Mischung beträgt.

11. Verfahren nach Anspruch 7, wobei der Gehalt des ersten Sprengmittels 2% bis 10%, vorzugsweise 5% bis 6%, bezogen auf das Gesamt-Trockengewicht aller Komponenten in Schritt (a), beträgt; und wobei der Gehalt des zweiten Sprengmittels 5% bis 10%, bezogen auf das Gesamt-Trockengewicht aller Komponenten in Schritt (c), beträgt.

12. Verfahren nach Anspruch 7, wobei der Gehalt des Gleitmittels 0,1% bis 1%, vorzugsweise 0,4% bis 0,5%, noch bevorzugter 0,48% bis 0,5%, Bezogen auf das Gesamt-Trockengewicht aller Komponenten in Schritt (c), beträgt.

13. Verfahren nach Anspruch 7, wobei das Verfahren ferner umfasst: (e) Beschichten eines in Schritt (d) erhaltenen Produkts.

14. Einzeldosisform, wobei die Einzeldosisform, bezogen auf das Gesamtgewicht der Einzeldosisform, umfasst:
10 mg, 20 mg oder 40 mg eines Wirkstoffs, wobei der Wirkstoff eine Verbindung der Formel (II) ist; 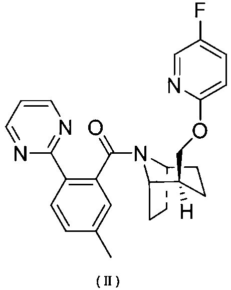
25 mg bis 150 mg mikrokristalline Cellulose;
50 mg bis 300 mg Lactose;
1 mg bis 15 mg Hypromellose;
10 mg bis 50 mg Croscarmellose-Natrium; und
0,5 mg bis 3 mg Magnesiumstearat,
wobei die Partikelgröße des Wirkstoffs D90 = 1 µm bis 20 µm beträgt und
wobei die Partikelgröße des Wirkstoffs mit einem Laser-Partikelgrößenanalysator gemessen wird.

15. Einzeldosisform, wobei die Einzeldosisform, bezogen auf das Gesamtgewicht der Einzeldosisform, umfasst:
8 mg bis 12 mg eines Wirkstoffs, wobei der Wirkstoff eine Verbindung der Formel (II) ist; 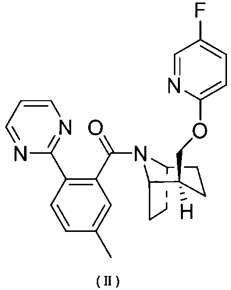
20 mg bis 30 mg mikrokristalline Cellulose;
46 mg bis 56 mg Lactose;
2 mg bis 4 mg Hypromellose;
5 mg bis 15 mg Croscarmellose-Natrium; und
0,3 mg bis 0,7 mg Magnesiumstearat,
wobei die Partikelgröße des Wirkstoffs D90 = 1 µm bis 20 µm beträgt, und
wobei die Partikelgröße des Wirkstoffs mit einem Laser-Partikelgrößenanalysator gemessen wird.

16. Einzeldosisform, wobei die Einzeldosisform, bezogen auf das Gesamtgewicht der Einzeldosisform, umfasst:
18 mg bis 22 mg eines Wirkstoffs, wobei der Wirkstoff eine Verbindung der Formel (II) ist; 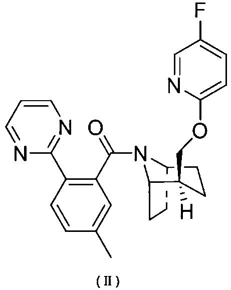
46 mg bis 56 mg mikrokristalline Cellulose;
97 mg bis 107 mg Lactose;
5 mg bis 7 mg Hypromellose;
15 mg bis 25 mg Croscarmellose-Natrium; und
0,8 mg bis 1,2 mg Magnesiumstearat,
wobei die Partikelgröße des Wirkstoffs D90 = 1 µm bis 20 µm beträgt, und
wobei die Partikelgröße des Wirkstoffs mit einem Laser-Partikelgrößenanalysator gemessen wird.

17. Einzeldosisform, wobei die Einzeldosisform, bezogen auf das Gesamtgewicht der Einzeldosisform, umfasst:
38 mg bis 42 mg eines Wirkstoffs, wobei der Wirkstoff eine Verbindung der Formel (II) ist; 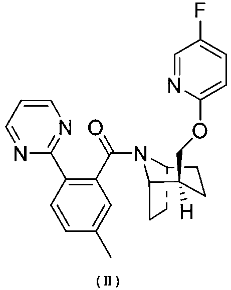
97 mg bis 107 mg mikrokristalline Cellulose;
199 mg bis 209 mg Lactose;
11 mg bis 13 mg Hypromellose;
35 mg bis 45 mg Croscarmellose-Natrium; und
1,8 mg bis 2,2 mg Magnesiumstearat,
wobei die Partikelgröße des Wirkstoffs D90 = 1 µm bis 20 µm beträgt, und
wobei die Partikelgröße des Wirkstoffs mit einem Laser-Partikelgrößenanalysator gemessen wird.

18. Feste pharmazeutische Formulierung gemäß einem der Ansprüche 1 bis 6 oder die Einzeldosisform gemäß einem der Ansprüche 14 bis 17, wobei die Verbindung der Formel (II) als Wirkstoff in kristalliner Form A oder kristalliner Form B vorliegt;
wobei die kristalline Form A ein Cu-Kα-Strahlungs-XRPD-Spektrum aufweist:
| Diffraktionswinkel 2θ | Relative Intensität (%) | Diffraktionswinkel 2θ | Relative Intensität (%) |
|---|---|---|---|
| 10.428 | 60.0 | 25.533 | 8.2 |
| 11.968 | 100.0 | 26.083 | 11.0 |
| 13.542 | 17.8 | 27.133 | 3.1 |
| 14.238 | 3.4 | 27.506 | 17.7 |
| 14.767 | 12.1 | 28.316 | 3.0 |
| 15.851 | 73.9 | 29.557 | 2.2 |
| 16.818 | 72.0 | 30.740 | 4.2 |
| 18.003 | 9.2 | 31.846 | 5.1 |
| 19.087 | 2.1 | 32.550 | 2.0 |
| 19.755 | 47.6 | 33.775 | 3.1 |
| 20.900 | 25.7 | 34.682 | 2.2 |
| 22.652 | 2.9 | 36.422 | 3.4 |
| 23.858 | 49.2 | 36.953 | 3.4 |
| 24.844 | 23.2 | 38.569 | 3.0 |
und wobei die kristalline Form B ein Cu-Kα-Strahlungs-XRPD-Spektrum aufweist:
| DiffraktionsWinkel 2θ | Relative Intensität (%) | DiffraktionsWinkel 20 | Relative Intensität (%) |
|---|---|---|---|
| 8.611 | 100.0 | 23.953 | 2.6 |
| 10.330 | 70.6 | 24.370 | 32.3 |
| 12.284 | 13.4 | 26.501 | 29.4 |
| 13.703 | 23.9 | 26.915 | 8.7 |
| 14.057 | 5.6 | 27.663 | 11.0 |
| 14.351 | 4.8 | 27.939 | 15.5 |
| 14.848 | 9.2 | 29.499 | 2.6 |
| 15.576 | 7.5 | 30.604 | 2.2 |
| 16.602 | 31.6 | 31.727 | 13.3 |
| 17.250 | 71.3 | 32.969 | 2.6 |
| 17.902 | 90.7 | 33.378 | 2.8 |
| 18.277 | 42.4 | 33.777 | 3.0 |
| 19.149 | 6.5 | 34.825 | 1.6 |
| 20.625 | 21.7 | 36.399 | 5.3 |
| 21.550 | 42.7 | 37.366 | 4.3 |
| 22.124 | 2.3 | 39.357 | 3.3 |
| 23.307 | 33.4 | | |

19. Einzeldosisform gemäß einem der Ansprüche 14 bis 17, wobei die Einzeldosisform eine Tablette oder eine Kapsel ist.

20. Feste pharmazeutische Formulierung gemäß einem der Ansprüche 1 bis 6 oder Einzeldosisform gemäß einem der Ansprüche 14 bis 17 zur Verwendung bei der Behandlung einer Orexin-assoziierten Erkrankung.

21. Feste pharmazeutische Formulierung oder Einzeldosisform zur Verwendung gemäß Anspruch 20, wobei die mit Orexin assoziierte Erkrankung Insomnie, chronisch obstruktive Lungenerkrankung, obstruktive Schlafapnoe, Somnolenz, Angstzustände, Zwangsstörungen, Panik, Nikotinabhängigkeit oder Essstörungen umfasst.

## Revendications

1. Formulation pharmaceutique solide, dans laquelle la formulation pharmaceutique solide comprend un ingrédient actif, et l'ingrédient actif est un composé représenté par la formule (II) ou un sel pharmaceutiquement acceptable de celui-ci, ou un mélange des deux ; 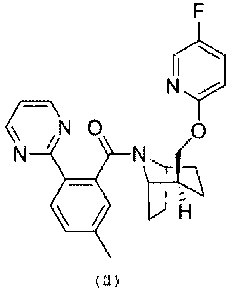
la taille de particule de l'ingrédient actif est D90 ≤ 35 µm,
dans laquelle la taille de particule de l'ingrédient actif est mesurée par un analyseur laser de distribution de taille de particule,
dans laquelle la teneur de l'ingrédient actif est de 1 % à 15 % sur la base du poids sec total de la formulation pharmaceutique solide ;
dans laquelle la formulation pharmaceutique solide comprend en outre un liant choisi dans le groupe consistant en l'hypromellose, l'hydroxypropylcellulose, la povidone, l'alginate de sodium, le carbopol, le poly(alcool vinylique) et une combinaison de ceux-ci, dans laquelle la teneur du liant est de 0,5 % à 10 % sur la base du poids sec total de la formulation pharmaceutique solide ;
dans laquelle la formulation pharmaceutique solide comprend en outre une charge choisie dans le groupe consistant en la cellulose microcristalline, le lactose, un complexe cellulose-lactose, l'amidon prégélatinisé, l'hydrogénophosphate de calcium, le carbonate de calcium et une combinaison de ceux-ci, dans laquelle la teneur de la charge est de 60 % à 90 % sur la base du poids sec total de la formulation pharmaceutique solide ;
dans laquelle la formulation pharmaceutique solide comprend en outre un agent de délitement choisi dans le groupe consistant en la croscarmellose sodique, l'hypromellose-K4M, la crospovidone, l'amidon carboxyméthylé sodique et une combinaison de ceux-ci, dans laquelle la teneur de l'agent de délitement est de 5 % à 15 % sur la base du poids sec total de la formulation pharmaceutique solide ; et
dans laquelle la formulation pharmaceutique solide comprend en outre un lubrifiant choisi dans le groupe consistant en le stéarate de magnésium, la poudre de talc, le monostéarate de glycérol, le stéarylfumarate de sodium et une combinaison de ceux-ci, dans laquelle la teneur du lubrifiant est de 0,1 % à 1 % sur la base du poids sec total de la formulation pharmaceutique solide.

2. Formulation pharmaceutique solide selon la revendication 1, dans laquelle la taille de particule de l'ingrédient actif est D90 ≤ 20 µm.

3. Formulation pharmaceutique solide selon la revendication 1, dans laquelle la formulation pharmaceutique solide est un comprimé, une gélule, une poudre, un granulé, une pilule en goutte ou un film, de préférence un comprimé.

4. Formulation pharmaceutique solide selon la revendication 1, dans laquelle la formulation pharmaceutique solide comprend les composants suivants sur la base du poids sec total de la formulation pharmaceutique solide :
a) l'ingrédient actif : ((1S,2R,5S)-2-(((5-fluoropyridin-2-yl)oxy)méthyl)-8-azabicyclo[3.2.1]octan-8-yl) (5-méthyl-2-(pyrimidin-2-yl)phényl)méthanone, ou un sel pharmaceutiquement acceptable de celle-ci, ou un mélange des deux, dans laquelle la teneur de l'ingrédient actif est de 4 % à 10 %, plus préférentiellement de 9,5 % à 10 % ;
b) une charge choisie dans le groupe consistant en la cellulose microcristalline, le lactose, un complexe cellulose-lactose, l'amidon prégélatinisé, l'hydrogénophosphate de calcium, le carbonate de calcium et une combinaison de ceux-ci, dans laquelle la teneur de la charge est de 73 % à 85 %, plus préférentiellement de 73 % à 82,5 % ;
c) un liant choisi dans le groupe consistant en l'hypromellose, l'hydroxypropylcellulose, la povidone, l'alginate de sodium, le carbopol, le poly(alcool vinylique) et une combinaison de ceux-ci, dans laquelle la teneur du liant est de 1,5 % à 3 % ;
d) un agent de délitement choisi dans le groupe consistant en la croscarmellose sodique, l'hypromellose-K4M, la crospovidone, l'amidon carboxyméthylé sodique et une combinaison de ceux-ci, dans laquelle la teneur de l'agent de délitement est de 5 % à 15 % ; et
e) un lubrifiant choisi dans le groupe consistant en le stéarate de magnésium, la poudre de talc, le monostéarate de glycérol, le stéarylfumarate de sodium et une combinaison de ceux-ci, dans laquelle la teneur du lubrifiant est de 0,4 % à 0,5 %, plus préférentiellement de 0,48 % à 0,5 % ;
dans laquelle la taille de particule de l'ingrédient actif est D90 ≤ 35 µm, ou D90 ≤ 30 µm, ou D90 ≤ 20 µm, ou D90 ≤ 10 µm, ou D90 = 1 µm à 30 µm, ou D90 = 1 µm à 20 µm, ou D90 = 1 µm à 10 µm, ou D90 = 10 µm à 20 µm.

5. Formulation pharmaceutique solide selon la revendication 1, dans laquelle la formulation pharmaceutique solide comprend les composants suivants sur la base du poids sec total de la formulation pharmaceutique solide :
a) l'ingrédient actif : ((1S,2R,5S)-2-(((5-fluoropyridin-2-yl)oxy)méthyl)-8-azabicyclo[3.2.1]octan-8-yl) (5-méthyl-2-(pyrimidin-2-yl)phényl)méthanone, ou un sel pharmaceutiquement acceptable de celle-ci, ou un mélange des deux, dans laquelle la teneur de l'ingrédient actif est de 4 % à 10 % ;
b) de la cellulose microcristalline à une teneur de 24 % à 27,5 % ;
c) du lactose à une teneur de 48,5 % à 56,5 % ;
d) de l'hypromellose-E5 à une teneur de 1,5 % à 3 % ;
e) de la croscarmellose sodique ou de l'hypromellose-K4M à une teneur de 5 % à 15 % ; et
f) du stéarate de magnésium à une teneur de 0,4 % à 0,5 % ; %
dans laquelle la taille de particule de l'ingrédient actif est D90 ≤ 35 µm, ou D90 ≤ 30 µm, ou D90 ≤ 20 µm, ou D90 ≤ 10 µm, ou D90 = 1 µm à 30 µm, ou D90 = 1 µm à 20 µm, ou D90 = 1 µm à 10 µm, ou D90 = 10 µm à 20 µm.

6. Formulation pharmaceutique solide selon la revendication 1, dans laquelle la formulation pharmaceutique solide comprend les composants suivants sur la base du poids sec total de la formulation pharmaceutique solide :
a) l'ingrédient actif : ((1S,2R,5S)-2-(((5-fluoropyridin-2-yl)oxy)méthyl)-8-azabicyclo[3.2.1]octan-8-yl) (5-méthyl-2-(pyrimidin-2-yl)phényl)méthanone, ou un sel pharmaceutiquement acceptable de celle-ci, ou un mélange des deux, dans laquelle la teneur de l'ingrédient actif est de 9,5 % à 10 % ;
b) de la cellulose microcristalline à une teneur de 24 % à 27,5 % ;
c) du lactose à une teneur de 48,5 % à 56,5 % ;
d) de l'hypromellose-E5 à une teneur de 1,5 % à 3 % ;
e) de la croscarmellose sodique ou de l'hypromellose-K4M à une teneur de 5 % à 15 % ; et
f) du stéarate de magnésium à une teneur de 0,48 % à 0,5 % ;
dans laquelle la taille de particule de l'ingrédient actif est D90 = 1 µm à 30 µm, ou D90 = 1 µm à 20 µm, ou D90 = 1 µm à 10 µm, ou D90 = 10 µm à 20 µm, et la formulation pharmaceutique solide est un comprimé.

7. Procédé de préparation de la formulation pharmaceutique solide selon la revendication 3, dans lequel la formulation pharmaceutique solide est un comprimé, dans lequel le procédé comprend les étapes suivantes :
(a) la réalisation d'une granulation humide après mélange d'une particule d'ingrédient actif, d'une charge, d'un liant et d'un premier agent de délitement ;
(b) le séchage d'un produit résultant obtenu à l'étape (a) ;
(c) le mélange à sec d'un produit résultant obtenu à l'étape (b), d'un second agent de délitement et d'un lubrifiant ; et
(d) la compression d'un produit résultant obtenu à l'étape (c) en comprimé ;
dans lequel l'ingrédient actif est la ((1S,2R,5S)-2-(((5-fluoropyridin-2-yl)oxy)méthyl)-8-azabicyclo[3.2.1]octan-8-yl) (5-méthyl-2-(pyrimidin-2-yl)phényl)méthanone, ou un sel pharmaceutiquement acceptable de celle-ci, ou un mélange des deux ; et
la taille de particule de la particule d'ingrédient actif est D90 ≤ 35µm.

8. Procédé selon la revendication 7, dans lequel la teneur de l'ingrédient actif est de 4 % à 10 % sur la base du poids sec total du mélange obtenu à l'étape (c).

9. Procédé selon la revendication 7, dans lequel la teneur de la charge est de 73 % à 82,5 % sur la base du poids sec total du mélange obtenu à l'étape (c).

10. Procédé selon la revendication 7, dans lequel la teneur du liant est de 1,5 % à 3 % sur la base du poids sec total du mélange obtenu à l'étape (c).

11. Procédé selon la revendication 7, dans lequel la teneur du premier agent de délitement est de 2 % à 10 %, plus préférentiellement de 5 % à 6 % sur la base du poids sec total de tous les composants à l'étape (a) ; et dans lequel la teneur du second agent de délitement est de 5 % à 10 % sur la base du poids sec total de tous les composants à l'étape (c).

12. Procédé selon la revendication 7, dans lequel la teneur du lubrifiant est de 0,1 % à 1 %, plus préférentiellement de 0,4 % à 0,5 %, plus préférentiellement de 0,48 % à 0,5 % sur la base du poids sec total de tous les composants à l'étape (c).

13. Procédé selon la revendication 7, dans lequel le procédé comprend en outre :
(e) l'enrobage d'un produit résultant obtenu à l'étape (d).

14. Forme posologique unitaire, dans laquelle, sur la base d'un poids total de la forme posologique unitaire, la forme posologique unitaire comprend :
10 mg, 20 mg ou 40 mg d'un ingrédient actif, dans laquelle l'ingrédient actif est un composé de formule (II) ; 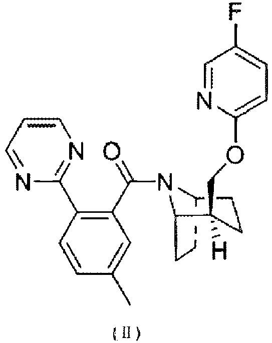
25 mg à 150 mg de cellulose microcristalline ;
50 mg à 300 mg de lactose ;
1 mg à 15 mg d'hypromellose ;
10 mg à 50 mg de croscarmellose sodique ; et 0,5 mg à 3 mg de stéarate de magnésium,
dans laquelle la taille de particule de l'ingrédient actif est D90 = 1 µm à 20 µm, et
dans laquelle la taille de particule de l'ingrédient actif est mesurée par un analyseur laser de distribution de taille de particule.

15. Forme posologique unitaire, dans laquelle, sur la base d'un poids total de la forme posologique unitaire, la forme posologique unitaire comprend :
8 mg à 12 mg d'un ingrédient actif, dans laquelle l'ingrédient actif est un composé de formule (II) ; 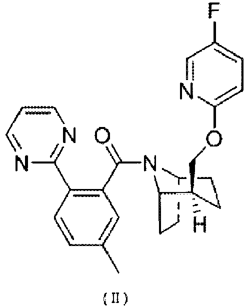
20 mg à 30 mg de cellulose microcristalline ;
46 mg à 56 mg de lactose ;
2 mg à 4 mg d'hypromellose ;
5 mg à 15 mg de croscarmellose sodique ; et
0,3 mg à 0,7 mg de stéarate de magnésium,
dans laquelle la taille de particule de l'ingrédient actif est D90 = 1 µm à 20 µm, et
dans laquelle la taille de particule de l'ingrédient actif est mesurée par un analyseur laser de distribution de taille de particule.

16. Forme posologique unitaire, dans laquelle, sur la base d'un poids total de la forme posologique unitaire, la forme posologique unitaire comprend :
18 mg à 22 mg d'un ingrédient actif, dans laquelle l'ingrédient actif est un composé de formule (II) ; 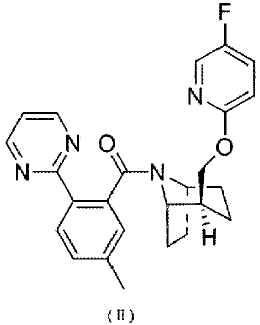
46 mg à 56 mg de cellulose microcristalline ;
97 mg à 107 mg de lactose ;
5 mg à 7 mg d'hypromellose ;
15 mg à 25 mg de croscarmellose sodique ; et
0,8 mg à 1,2 mg de stéarate de magnésium,
dans laquelle la taille de particule de l'ingrédient actif est D90 = 1 µm à 20 µm, et
dans laquelle la taille de particule de l'ingrédient actif est mesurée par un analyseur laser de distribution de taille de particule.

17. Forme posologique unitaire, dans laquelle, sur la base d'un poids total de la forme posologique unitaire, la forme posologique unitaire comprend :
38 mg à 42 mg d'un ingrédient actif, dans laquelle l'ingrédient actif est un composé de formule (II) ; 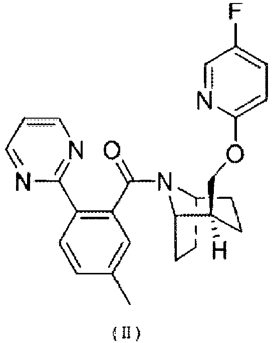
97 mg à 107 mg de cellulose microcristalline ;
199 mg à 209 mg de lactose ;
11 mg à 13 mg d'hypromellose ;
35 mg à 45 mg de croscarmellose sodique ; et
1,8 mg à 2,2 mg de stéarate de magnésium,
dans laquelle la taille de particule de l'ingrédient actif est D90 = 1 µm à 20 µm, et
dans laquelle la taille de particule de l'ingrédient actif est mesurée par un analyseur laser de distribution de taille de particule.

18. Formulation pharmaceutique solide selon l'une des revendications 1 à 6 ou forme posologique unitaire selon l'une des revendications 14 à 17, dans laquelle le composé de formule (II) en tant que principe actif existe sous une forme cristalline A ou sous une forme cristalline B ;
dans laquelle la forme cristalline A présente un spectre XRPD de radiation K α du Cu :
| **Angle de diffraction 2θ** | **Intensité relative (%)** | **Angle de diffraction 2θ** | **Intensité relative (%)** |
|---|---|---|---|
| 10,428 | 60, 0 | 25,533 | 8,2 |
| 11, 968 | 100,0 | 26, 083 | 11,0 |
| 13,542 | 17, 8 | 27,133 | 3,1 |
| 14,238 | 3,4 | 27,506 | 17,7 |
| 14,767 | 12,1 | 28,316 | 3,0 |
| 15,851 | 73, 9 | 29,557 | 2,2 |
| 16, 818 | 72, 0 | 30,740 | 4,2 |
| 18, 003 | 9,2 | 31,846 | 5,1 |
| 19, 087 | 2,1 | 32, 550 | 2,0 |
| 19, 755 | 47, 6 | 33,775 | 3,1 |
| 20, 900 | 25,7 | 34, 682 | 2,2 |
| 22, 652 | 2, 9 | 36, 422 | 3, 4 |
| 23, 858 | 49, 2 | 36, 953 | 3, 4 |
| 24, 844 | 23,2 | 38,569 | 3, 0 |
; et
dans laquelle la forme cristalline B présente un spectre XRPD de radiation Kα du Cu :
| **Angle de diffraction 2θ** | **Intensité relative (%)** | **Angle de diffraction 20** | **Intensité relative (%)** |
|---|---|---|---|
| 8,611 | 100,0 | 23, 953 | 2, 6 |
| 10,330 | 70, 6 | 24,370 | 32,3 |
| 12,284 | 13,4 | 26, 501 | 29, 4 |
| 13,703 | 23,9 | 26, 915 | 8, 7 |
| 14,057 | 5, 6 | 27, 663 | 11,0 |
| 14,351 | 4, 8 | 27, 939 | 15,5 |
| 14, 848 | 9,2 | 29, 499 | 2, 6 |
| 15,576 | 7,5 | 30, 604 | 2,2 |
| 16, 602 | 31, 6 | 31,727 | 13,3 |
| 17,250 | 71,3 | 32,969 | 2, 6 |
| 17, 902 | 90, 7 | 33,378 | 2,8 |
| 18, 277 | 42,4 | 33,777 | 3, 0 |
| 19,149 | 6,5 | 34, 825 | 1, 6 |
| 20, 625 | 21,7 | 36, 399 | 5,3 |
| 21,550 | 42,7 | 37,366 | 4,3 |
| 22, 124 | 2,3 | 39, 357 | 3,3 |
| 23,307 | 33,4 | | |

19. Forme posologique unitaire selon l'une des revendications 14 à 17, dans laquelle la forme posologique unitaire est un comprimé ou une gélule.

20. Formulation pharmaceutique solide selon l'une des revendications 1 à 6, ou forme posologique unitaire selon l'une des revendications 14 à 17, pour une utilisation dans le traitement d'une maladie associée à l'orexine.

21. Formulation pharmaceutique solide ou forme posologique unitaire pour une utilisation selon la revendication 20, dans laquelle la maladie associée à l'orexine inclut l'insomnie, la bronchopneumopathie chronique obstructive, l'apnée obstructive du sommeil, la somnolence, l'anxiété, le trouble obsessionnel compulsif, les crises de panique, la dépendance à la nicotine ou les troubles de l'alimentation.
